(19) Europäisches Patentamt / European Patent Office / Office européen des brevets

(11) **EP 4 215 110 A1**

(12) **EUROPEAN PATENT APPLICATION**
published in accordance with Art. 153(4) EPC

(43) Date of publication:
26.07.2023 Bulletin 2023/30

(21) Application number: 21884653.3

(22) Date of filing: 31.08.2021

(51) International Patent Classification (IPC):
**A61B 5/053** $^{(2021.01)}$

(52) Cooperative Patent Classification (CPC):
**A61B 5/00; A61B 5/053; A61B 5/0537;
G01G 19/50**

(86) International application number:
**PCT/CN2021/115493**

(87) International publication number:
**WO 2022/088921 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 30.10.2020 CN 202011193817

(71) Applicant: Huawei Technologies Co., Ltd.
Shenzhen, Guangdong 518129 (CN)

(72) Inventors:
• ZHAO, Shuai
Shenzhen, Guangdong 518129 (CN)
• REN, Huichao
Shenzhen, Guangdong 518129 (CN)

• YANG, Bin
Shenzhen, Guangdong 518129 (CN)
• YE, Jilong
Shenzhen, Guangdong 518129 (CN)
• GUO, Jianhua
Shenzhen, Guangdong 518129 (CN)
• WU, Lian
Shenzhen, Guangdong 518129 (CN)
• MA, Shanshan
Shenzhen, Guangdong 518129 (CN)
• LI, Junjin
Shenzhen, Guangdong 518129 (CN)

(74) Representative: Huawei European IPR
Huawei Technologies Duesseldorf GmbH
Riesstraße 25
80992 München (DE)

(54) **BODY COMPOSITION MEASUREMENT METHOD AND APPARATUS**

(57) A human body composition measurement method and an apparatus are provided, to obtain accurate whole human body compositions or accurate segmental body compositions of a measurement object by a body fat measurement device (210). The human body composition measurement method includes: The body fat measurement device (210) obtains measurement information of the measurement object, where the measurement information includes weight information and impedance information, and the impedance information is obtained by measuring a first segment of the measurement object; and the body fat measurement device (210) determines second segmental body composition information of the measurement object based on the weight information, the impedance information, and first segmental body composition information of the measurement object.

EP 4 215 110 A1

**Measurement information**

- **Weight information W**
- **First impedance $Z_1$**
- **Second impedance $Z_2$**

**First type of personal information**

- **Height information H**
- **Gender information G**
- **Age information Age**

702: The four-electrode body fat scale determines whole-body compositions

701: A four-electrode body fat scale determines leg compositions

**Whole-body composition**

- **Whole-body fat mass FM**
- **Whole-body muscle mass MM**

**Leg compositions**

- **Left leg fat mass LLFM**
- **Left leg muscle mass LLMM**
- **Right leg fat mass RLFM**
- **Right leg muscle mass RLMM**

704: The four-electrode body fat scale determines trunk compositions

**Trunk compositions**

- **Trunk fat mass FM**
- **Trunk muscle mass MM**

703: The four-electrode body fat scale determines arm compositions

**Arm compositions**

- **Left arm fat mass LAFM**
- **Left arm muscle mass LAMM**
- **Right arm fat mass RAFM**
- **Right arm muscle mass RAMM**

FIG. 7

# Description

## CROSS-REFERENCE TO RELATED APPLICATIONS

This application claims priority to Chinese Patent Application No. 202011193817.7, filed with the China National Intellectual Property Administration on October 30, 2020 and entitled "HUMAN BODY COMPOSITION MEASUREMENT METHOD AND APPARATUS", which is incorporated herein by reference in its entirety.

## TECHNICAL FIELD

This application relates to the field of terminal technologies, and in particular, to a human body composition measurement method and an apparatus.

## BACKGROUND

Human body compositions include contents of fat, muscle, protein, minerals, and other composition in a human body. The human body compositions can be used to evaluate development, maturity, and aging statuses of the human body, and are of great significance in monitoring children's development, guiding body building and weight loss, providing medical care, and the like. The most commonly used method for human body composition detection is bioelectrical impedance analysis (bioelectrical impedance analysis, BIA). Based on a principle that various compositions such as the fat and the muscle in the human body have different conductivities, human body impedance is determined by passing a weak current through the human body, and then contents of the body compositions in the human bodies are calculated based on the human body impedance.

A body fat scale may determine contents of body compositions in a human body by using the BIA. Specifically, the body fat scale emits, by using an electrode plate/a conductive film, a weak current that cannot be perceived by the human body, and the current flows from one foot of the human body to the other foot of the human body, to form a closed loop. Therefore, the body fat scale can determine lower-body impedance of the human body, but accuracy of a body composition result obtained based on the lower-body impedance is low.

## SUMMARY

This application provides a human body composition measurement method and an apparatus, to obtain accurate whole human body compositions or accurate segmental body compositions of a measurement object by a body fat measurement device.

According to a first aspect, this application provides a human body composition measurement method, including: A body fat measurement device obtains measurement information of a measurement object, where the measurement information includes weight information and impedance information, and the impedance information is obtained by measuring a first segment of the measurement object; the body fat measurement device determines first segmental body composition information of the measurement object based on the weight information and the impedance information; and the body fat measurement device determines second segmental body composition information of the measurement object based on the weight information, the impedance information, and the first segmental body composition information of the measurement object.

In the foregoing technical solution, the body fat measurement device obtains the impedance information of the first segment by measuring the first segment of the measurement object, and then determines the accurate first segmental body composition information of the measurement object based on the weight information and the impedance information of the measurement object. The second segmental body composition information of the measurement object is determined based on the first segmental body composition information of the measurement object together with the weight information and the impedance information of the measurement object. This is conducive to obtaining the accurate second segmental body composition information of the measurement object.

In a possible implementation, the impedance information includes first impedance and second impedance, the first impedance is obtained by measuring the first segment by using a measurement signal at a first frequency, the second impedance is obtained by measuring the first segment by using a measurement signal at a second frequency, and the first frequency is less than the second frequency.

In a possible implementation, the first frequency is 50 kHz, and the second frequency is 250 kHz.

In a possible implementation, the second segmental body composition information of the measurement object satisfies the following relational expression:

$$AM=\alpha_1 Z_1+\alpha_2 Z_2+\alpha_3 W+\alpha_4 H+\alpha_5 Age+\alpha_6 G+\alpha_7 LM+\alpha_0,$$

where

AM is the second segmental body composition information, $Z_1$ is the first impedance, $\alpha_1$ is a weighting coefficient of the first impedance, $Z_2$ is the second impedance, $\alpha_2$ is a weighting coefficient of the second impedance, W is the weight information of the measurement object obtained by the body fat measurement device, $\alpha_3$ is a weighting coefficient of the weight information, H is height information of the measurement object obtained by the body fat measurement device, $\alpha_4$ is a weighting coefficient of the height information, Age is age information of the measurement object obtained by the body fat measurement device, as is a weighting coefficient of the age information, G is gender information of the measurement object obtained by the body fat measurement device, a value of G is 1 or 0, $\alpha_6$ is a weighting coefficient of the gender information, LM is the first segmental body composition information, $\alpha_7$ is a weighting coefficient of the first segmental body composition information, and ao is a constant.

In a possible implementation, the method further includes: The body fat measurement device determines whole-body composition information of the measurement object based on the weight information, a weighting coefficient of the weight information, the impedance information, and a weighting coefficient of the impedance information; and the body fat measurement device determines third segmental body composition information of the measurement object based on the whole-body composition information, the first segmental body composition information, and the second segmental body composition information of the measurement object.

In a possible implementation, the body fat measurement device is a four-electrode body fat scale, the first segmental body composition information includes left leg composition information and/or right leg composition information, the second segmental body composition information includes left arm composition information and/or right arm composition information, and the third segmental body composition information includes trunk composition information.

In a possible implementation, the method further includes: determining a limb balance indicator of the measurement object based on the whole-body composition information, the first segmental body composition information, the second segmental body composition information, and the third segmental body composition information.

In a possible implementation, the method further includes: The body fat measurement device displays any one or more of the following on a display interface of the body fat measurement device: the first segmental body composition information, the second segmental body composition information, the third segmental body composition information, the whole-body composition information, the limb balance indicator, and an exercise suggestion corresponding to the limb balance indicator of the measurement object.

In a possible implementation, the body fat measurement device is a wearable device, the measurement information further includes a measurement manner, and the measurement manner is corresponding to the first segment; and that the body fat measurement device determines second segmental body composition information of the measurement object based on the weight information, the impedance information, and the first segmental body composition information of the measurement object includes: The wearable device inputs the weight information, the impedance information, and the first segmental body composition information of the measurement object into a body composition algorithm model corresponding to the measurement manner, to obtain the second segmental body composition information of the measurement object.

In the foregoing technical solution, the body fat measurement device is a wearable device, and the body composition algorithm model corresponding to the measurement manner is disposed in the wearable device. Then, a specific body composition algorithm model into which a measurement result is input is determined based on the measurement manner determined when the measurement object wears the wearable device.

In addition, when the wearable device is used, the wearable device may detect motion information (for example, a current day's exercise amount and current exercise intensity), sleep information (for example, sleep quality and sleep duration), diet information, heart information (for example, a heart rate), and the like of the measurement object. Therefore, input parameters of the body composition algorithm model may include the weight information and the impedance information of the measurement object and the first segmental body composition information of the measurement object, and also include the motion information, the sleep information, the diet information, the heart information, and the like. This can further improve accuracy of the determined second segmental body composition information of the measurement object.

In a possible implementation, before the body fat measurement device obtains the measurement information of the measurement object, the method further includes: The body fat measurement device obtains contact information between the measurement object and the body fat measurement device, where the contact information includes two-hand contact information and/or two-foot contact information between the body fat measurement device and the measurement object; and the body fat measurement device determines that the contact information of the measurement object meets a preset requirement.

In a possible implementation, the two-foot contact information includes two-foot spacing information, two-foot pressure information, and standing location information; and that the two-foot contact information meets a preset requirement includes at least one of the following: The two-foot spacing information indicates that a two-foot spacing of the measurement object is greater than a distance threshold; the two-foot pressure information indicates that a pressure difference between two feet of the measurement object is not greater than a pressure difference threshold; and the standing location information indicates that the two feet of the measurement object stand in a first specified region of the body fat measurement device.

In a possible implementation, the two-hand contact information includes handle holding area information, handle rope length information, and handle holding location information; and that the two-hand contact information meets a preset requirement includes at least one of the following: The handle holding area information indicates that a contact area between two hands of the measurement object and a handle of the body fat measurement device is greater than an area threshold; the handle rope length information indicates that a handle rope length of the body fat measurement device falls within a preset rope length range; and the handle holding location information indicates that the two hands of the measurement object hold in a second specified region of the handle of the body fat measurement device.

In a possible implementation, the body fat measurement device is a four-electrode body fat scale, and the contact information includes the two-foot contact information; or the body fat measurement device is an eight-electrode body fat scale, and the contact information includes the two-hand contact information and the two-foot contact information.

In the foregoing technical solution, before obtaining the measurement information of the measurement object, the body fat measurement device may further first obtain the two-hand contact information and/or the two-foot contact information of the measurement object, to ensure that the measurement object maintains a correct measurement posture, and improve measurement accuracy of the body fat measurement device.

According to a second aspect, this application provides a body fat measurement device. The body fat measurement device includes one or more processors, a memory, a sensor, a transceiver, and one or more computer programs. The one or more computer programs are stored in the memory, the one or more computer programs include instructions, and when the instructions are invoked and executed by the one or more processors, the body fat measurement device is enabled to perform the method according to any one of the first aspect or the possible implementations of the first aspect.

According to a third aspect, this application provides a computer-readable storage medium. The computer-readable storage medium stores a computer program or instructions, and when the computer program or instructions are executed by a computer, the method according to any one of the first aspect or the possible implementations of the first aspect is implemented.

According to a fourth aspect, this application provides a computer program product. The computer program product includes a computer program or instructions, and when the computer program or instructions are executed by a computer, the method according to any one of the first aspect or the possible implementations of the first aspect is implemented.

For technical effects that can be achieved in any one of the second aspect to the fourth aspect, reference may be made to the foregoing descriptions of the beneficial effects in the first aspect. Details are not described herein again.

**BRIEF DESCRIPTION OF DRAWINGS**

FIG. 1 is a schematic diagram of a structure of a human body according to this application;
FIG. 2 is a diagram of a system architecture to which a human body composition measurement method is applicable according to this application;
FIG. 3 is a schematic diagram of a structure of a body fat measurement device according to this application;
FIG. 4 is a schematic diagram of a four-electrode body fat scale according to this application;
FIG. 5 is a schematic diagram of a principle of detecting human body impedance by a four-electrode body fat scale according to this application;
FIG. 6 is a schematic flowchart of a human body composition measurement method according to this application;
FIG. 7 is a schematic flowchart of another human body composition measurement method according to this application;
FIG. 8 is a schematic flowchart of determining a balance result of a detected person according to this application;
FIG. 9 is a diagram of a display interface according to this application;
FIG. 10 is a schematic diagram of a detection procedure for determining segmental body compositions of a detected person according to this application;
FIG. 11 is a schematic flowchart of a first adjustment method according to this application;
FIG. 12 is a schematic diagram in which a body fat measurement device is in contact with two feet of a detected person according to this application;
FIG. 13 is a schematic flowchart of a second adjustment method according to this application;
FIG. 14 is another schematic diagram in which a body fat measurement device is in contact with two feet of a detected person according to this application;
FIG. 15 is a schematic flowchart of determining whether a detected person stands in a first specified region according to this application;
FIG. 16 is a schematic diagram of a location at which two feet of a detected person stand on a four-electrode body fat scale according to this application;
FIG. 17 is a schematic diagram of an eight-electrode body fat scale according to this application;
FIG. 18 is a schematic diagram of a principle of detecting human body impedance by an eight-electrode body fat scale according to this application;
FIG. 19 is a schematic flowchart of a third adjustment method according to this application;
FIG. 20 is a schematic diagram in which a body fat measurement device is in contact with two hands of a detected person according to this application;
FIG. 21 is a schematic flowchart of a fourth adjustment method according to this application;
FIG. 22 is a schematic diagram of determining a handle rope length according to this application;

FIG. 23 is a schematic flowchart of determining whether a detected person holds in a second specified region according to this application;

FIG. 24 is a schematic flowchart of a fifth adjustment method according to this application;

FIG. 25 is a schematic diagram of a structure of a smart band according to this application;

FIG. 26 is a schematic diagram in which a detected person wears a smart band according to this application;

FIG. 27 is a schematic flowchart of still another human body composition measurement method according to this application;

FIG. 28 is a schematic flowchart of yet another human body composition measurement method according to this application; and

FIG. 29 is a schematic diagram of a structure of a body fat measurement device according to this application.

## DESCRIPTION OF EMBODIMENTS

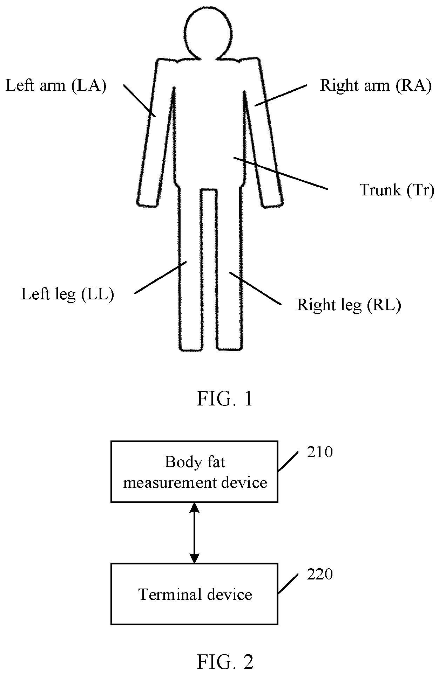

For ease of describing embodiments of this application, human body segments in embodiments of this application are first described. FIG. 1 is a schematic diagram of a structure of a human body according to this application. The human body includes a plurality of segments, which are specifically a left arm (or referred to as a left upper limb, full name in English: left arm, LA for short), a right arm (or referred to as a right upper limb, full name in English: right arm, RA for short), a left leg (or referred to as a left lower limb, full name in English: left leg, LL for short), a right leg (or referred to as a right lower limb, full name in English: right leg, RL for short), and a trunk (full name in English: trunk, Tr for short).

For example, human body compositions may include fat, muscle, skeletal muscle, and protein, and human body composition information (or human body composition contents) may include fat mass, a fat rate, muscle mass, a muscle rate, skeletal muscle mass, a skeletal muscle rate, protein mass, a protein rate, bone salt mass, and a bone salt rate. A relationship between fat mass and a fat rate is used as an example herein. The fat mass is mass (mass) of fat, and the fat rate is a rate (rate) of fat mass to body mass. The following describes the human body composition information by using human body composition mass such as fat mass and muscle mass as an example.

A whole body or each segment of the whole body is combined with the human body composition information. Whole-body composition information may include whole-body fat mass (full name in English: fat mass, FM for short) and whole-body muscle mass (full name in English: muscle mass, MM for short).

Segmental body composition information of the human body may include left arm fat mass (full name in English: left arm fat mass, LAFM for short), right arm fat mass (full name in English: right arm fat mass, RAFM for short), left leg fat mass (full name in English: left leg fat mass, LLFM for short), right leg fat mass (full name in English: right leg fat mass, RLFM for short), trunk fat mass (full name in English: trunk fat mass, TrFM for short), left arm muscle mass (full name in English: left arm muscle mass, LAMM for short), right arm muscle mass (full name in English: right arm muscle mass, RAMM for short), left leg muscle mass (full name in English: left leg muscle mass, LLMM for short), right leg muscle mass (full name in English: right leg muscle mass, RLMM for short), trunk muscle mass (full name in English: trunk muscle mass, TrMM for short), and the like.

Because contents of fat, muscle, and other compositions in different human body segments are different and conductivities of different compositions are different, human body segments are corresponding to respective impedance. Based on this, the impedance of the human body segments may be detected by using BIA, to derive and calculate the whole-body composition information of the human body and/or the segmental body composition information of the human body.

FIG. 2 is a diagram of a system architecture to which a human body composition measurement method is applicable according to this application. The system architecture includes a body fat measurement device 210 and a terminal device 220.

In an implementation, the body fat measurement device 210 obtains measurement information by using a weak current in a human body, and determines body composition information of a detected person based on the measurement information. A detecting person or the detected person may further enter a first type of personal information of the detected person into the terminal device 220. The first type of personal information is, for example, a gender, an age, a height, and a bust/waist/hip of the detected person. The terminal device 220 sends the first type of personal information of the detected person to the body fat measurement device 210. The body fat measurement device 210 determines the human body composition information of the detected person based on the measurement information and the first type of personal information of the detected person. In this manner, with reference to the first type of personal information of the detected person, accuracy of determining the human body composition information can be improved.

Further, the body fat measurement device 210 may display the body composition information of the detected person, or send the human body composition information of the detected person to the terminal device 220, so that the terminal device 220 displays the body composition information of the detected person.

Specifically, in a process in which the body fat measurement device 210 or the terminal device 220 displays the body composition information of the detected person, the body fat measurement device 210 or the terminal device 220 may directly display the human body composition information of the detected person, for example, contents of whole-body compositions and/or contents of corresponding segmental body compositions of the detected person. Alternatively, the body fat measurement device 210 or the terminal device 220 may further calculate a limb balance indicator of the detected person, and display the limb balance indicator of the detected person separately or together with the human body composition information on a display interface of the body fat measurement device 210 or the terminal device 220.

In another implementation, the body fat measurement device 210 obtains measurement information by using a weak current in a human body, and sends the measurement information of a detected person to the terminal device 220. The terminal device 220 determines human body composition information of the detected person based on the measurement information. A detecting person or the detected person may further enter a first type of personal information of the detected person into the terminal device 220. The terminal device 220 determines the human body composition information of the detected person based on the measurement information and the first type of personal information of the detected person. In this manner, with reference to the first type of personal information of the detected person, accuracy of determining the human body composition information can be improved.

Further, the terminal device 220 may directly display the human body composition information of the detected person, or may calculate a limb balance indicator of the detected person, and display the limb balance indicator of the detected person separately or together with the human body composition information on a display interface of the terminal device 220.

For example, the body fat measurement device 210 may be a body fat scale or a wearable device. The body fat scale is, for example, a four-electrode body fat scale or an eight-electrode body fat scale. The wearable device may be, for example, a smart band, a smartwatch, or a smart anklet.

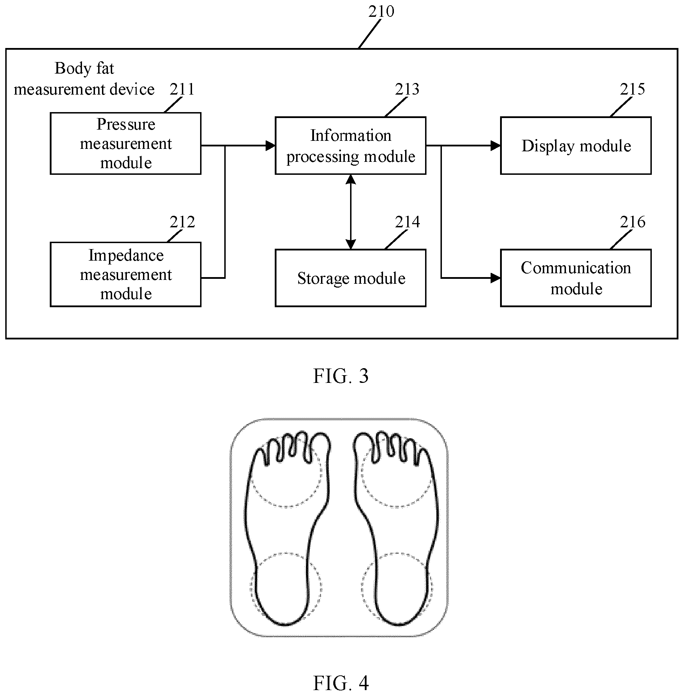

FIG. 3 is an example of a schematic diagram of a structure of a body fat measurement device 210. The body fat measurement device 210 includes: an impedance measurement module 212, configured to measure impedance information of a detected person by using a measurement signal, to obtain measurement information; an information processing module 213, configured to perform processing and logic control on the measurement signal; a storage module 214, configured to store the measurement information; a display module 215, configured to display a measurement result and interact with a user; and a communication module 216, configured to perform information transmission with the terminal device 220. In addition, if the body fat measurement device 210 is a body fat scale, the body fat measurement device 210 may further include a pressure measurement module 211, configured to measure information about pressure exerted by the detected person on the body fat measurement device 210.

An embodiment of this application provides a human body composition measurement method. A body fat measurement device determines segment impedance information of a measurement object, determines corresponding segmental body composition information based on the segment impedance information of the measurement object, and further determines whole-body composition information of the measurement object based on the segmental body composition information.

The method provided in this embodiment of this application is applicable to body composition measurement of a human body, and is also applicable to body composition measurement of another organism in some cases. In other words, the measurement object may be a detected person, or may be another organism. In the following embodiments, determining human body composition information is used as an example for description, that is, that the measurement object is a person is used as an example for description.

As shown in FIG. 1, segment impedance of the detected person include lower-body impedance (an impedance between two legs), upper-body impedance (impedance between two arms), left-body impedance (impedance between a left upper limb, a trunk, and a left lower limb), right-body impedance (impedance between a right upper limb, the trunk, and a right lower limb), right-upper left-lower body impedance (impedance between the right upper limb, the trunk, and the left lower limb), and left-upper right-lower body impedance (impedance between the left upper limb, the trunk, and the right lower limb).

The method provided in this embodiment of this application may be implemented in the body fat measurement device 210 shown in FIG. 2. The body fat measurement device 210 may be, for example, a four-electrode body fat scale, an eight-electrode body fat scale, or a wearable device. Based on different measurement principles of different devices, the following describes the human body composition measurement method in this application in different cases.

In a first case, the body fat measurement device is a four-electrode body fat scale.

A measurement principle of the four-electrode body fat scale is first described as follows.

FIG. 4 is an example of a schematic diagram of a four-electrode body fat scale according to this application. Four electrodes (as denoted by four dashed circles in FIG. 4) are disposed in a scale body of the four-electrode body fat scale. Two feet of a detected person step on the scale body, front soles of the two feet of the detected person are in contact with two electrodes, and rear soles of the two feet of the detected person are in contact with the other two electrodes.

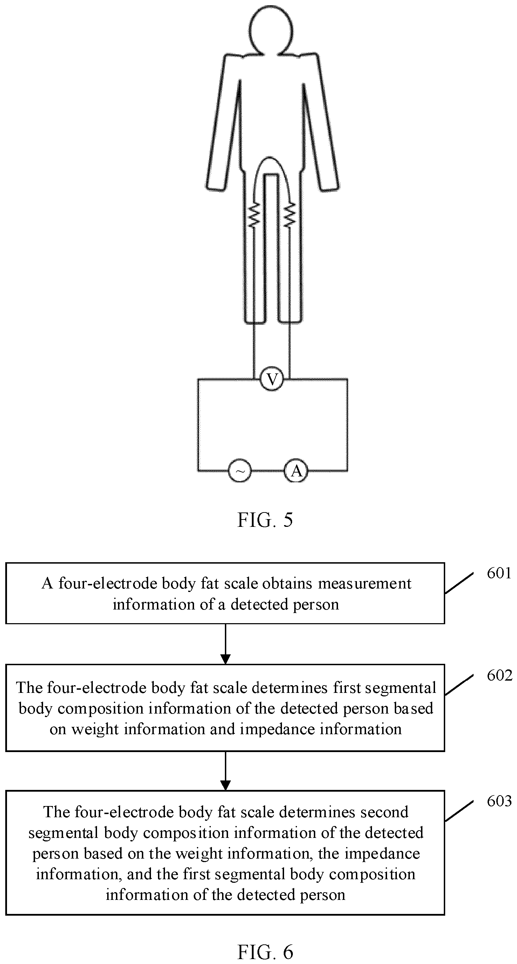

FIG. 5 is an example of a schematic diagram of a principle of detecting human body impedance by a four-electrode body fat scale according to this application. The four-electrode body fat scale may conduct an alternating current on the two electrodes in contact with the front soles, where the current forms a current loop between two legs; measure a voltage on the two electrodes in contact with the rear soles; and determine lower-body impedance of the detected person based on the measured voltage.

Based on the descriptions of FIG. 4 and FIG. 5, FIG. 6 is a schematic flowchart of a human body composition measurement method according to this application.

Step 601: The four-electrode body fat scale obtains measurement information of the detected person.

The measurement information of the detected person includes weight information and impedance information of the detected person, where the impedance information is obtained by the four-electrode body fat scale by measuring the lower-body impedance of the detected person. Specifically, the four-electrode body fat scale measures a lower body of the detected person by using a measurement signal, to obtain the lower-body impedance of the detected person, and determines the impedance information based on the lower-body impedance. The measurement signal may be an alternating current.

The four-electrode body fat scale may use a single-frequency or multi-frequency alternating current to measure the lower-body impedance of the detected person. If using a multi-frequency alternating current to measure the lower-body impedance of the detected person, the four-electrode body fat scale may measure lower-body impedance corresponding to an alternating current at each frequency, and use the lower-body impedance corresponding to the alternating current at each frequency to form the impedance information of the detected person.

In an implementation, the four-electrode body fat scale uses a dual-frequency alternating current to measure the impedance information of the detected person. Specifically, the four-electrode body fat scale uses an alternating current at a first frequency to measure the lower body of the detected person, to obtain first impedance $Z_1$, uses an alternating current at a second frequency to measure the lower body of the detected person, to obtain second impedance $Z_2$, and uses the first impedance $Z_1$ and the second impedance $Z_2$ to form the impedance information of the detected person. The first frequency is less than the second frequency.

For example, the detected person stands on the four-electrode body fat scale shown in FIG. 4. The four-electrode body fat scale first conducts the alternating current at the first frequency on the two electrodes in contact with the front soles, measures a voltage on the two electrodes in contact with the rear soles, and uses determined lower-body impedance as the first impedance $Z_1$. Then, the four-electrode body fat scale conducts the alternating current at the second frequency on the two electrodes in contact with the front soles, measures a voltage on the two electrodes in contact with the rear soles, and determines determined lower-body impedance as the second

impedance $Z_2$.

In this application, a high-frequency alternating current may penetrate human body cells, and is mainly used to measure intracellular compositions. A low-frequency alternating current has a weak capability of penetrating human body cells, and is mainly used to measure extracellular compositions. Values of the first frequency and the second frequency both fall within a range of 1 kHz to 1000 kHz. For example, the first frequency is 50 kHz and the second frequency is 250 kHz, or the first frequency is 20 kHz and the second frequency is 80 kHz.

In addition, the four-electrode body fat scale may further measure a weight of the detected person to obtain the weight information W of the detected person. For example, the pressure measurement module 211 in FIG. 3 may be used to measure the weight information of the detected person.

Step 602: The four-electrode body fat scale determines first segmental body composition information of the detected person based on the weight information and the impedance information.

A first segment may be legs, including a left leg and a right leg.

In a first implementation, the four-electrode body fat scale determines leg composition information of the detected person based on the weight information and the impedance information of the detected person, and may specifically determine left leg composition information and right leg composition information of the detected person.

In an example, the four-electrode body fat scale uses the weight information and the impedance information of the detected person as input parameters, and inputs the input parameters into a trained first model. The model outputs the left leg composition information and the right leg composition information of the detected person. Model training may be performed in the four-electrode body fat scale, or may be performed in another device.

In another example, the four-electrode body fat scale determines the leg composition information by using a multivariate linear regression calculation method. The leg composition information satisfies a relational expression: $LM=\beta_1 Z_1+\beta_2 Z_2+\beta_3 W+\beta_0$, where LM (leg mass) is the leg composition information, $\beta_1$, $\beta_2$, $\beta_3$, and $\beta_0$ may be weighting coefficients determined experimentally, $Z_1$ is the first impedance, $Z_2$ is the second impedance, and W is the weight information.

Specifically, the left leg composition information satisfies a relational expression: $LLM=\beta_{L1} Z_1+\beta_{L2} Z_2+\beta_{L3} W+\beta_{L0}$, where LLM (left leg mass) is the left leg composition information, and $\beta_{L1}$, $\beta_{L2}$, $\beta_{L3}$, and $\beta_{L0}$ may be weighting coefficients determined experimentally. Further, left leg fat mass is $LLFM=\beta_{LF1} Z_1+\beta_{LF2} Z_2+\beta_{LF3} W+\beta_{LF0}$, and left leg muscle mass is $LLMM=\beta_{LM1} Z_1+\beta_{LM2} Z_2+\beta_{LM3} W+\beta_{LM0}$.

The right leg composition information satisfies a relational expression: $RLM=\beta_{R1} Z_1+\beta_{R2} Z_2+\beta_{R3} W+\beta_{R0}$,

where RLM (right leg mass) is the right leg composition information, and $\beta_{R1}$, $\beta_{R2}$, $\beta_{R3}$, and $\beta_{R0}$ may be weighting coefficients determined experimentally. Further, right leg fat mass is $RLFM=\beta_{RF1}Z_1+\beta_{RF2}Z_2+\beta_{RF3}W+\beta_{RF0}$, and right leg muscle mass is $RRMM=\beta_{RM1}Z_1+\beta_{RM2}Z_2+\beta_{RM3}W+\beta_{RM0}$.

To enable the four-electrode body fat scale to more accurately obtain the leg composition information of the detected person, this application provides a second implementation: The four-electrode body fat scale determines leg composition information of the detected person with reference to a first type of personal information of the detected person.

Specifically, the four-electrode body fat scale determines the leg composition information of the detected person based on the weight information and the impedance information of the detected person and with reference to the first type of personal information of the detected person, for example, a gender, an age, a height, and a bust/waist/hip, and may specifically determine left leg composition information and right leg composition information of the detected person.

In an example, the four-electrode body fat scale uses the weight information, the impedance information, the gender, the age, the height, and the bust/waist/hip of the detected person as input parameters, and inputs the input parameters into a trained second model. The model outputs the left leg composition information and the right leg composition information of the detected person. Model training may be performed in the four-electrode body fat scale, or may be performed in another device.

In another example, the four-electrode body fat scale determines the leg composition information by using a multivariate linear regression calculation method. The leg composition information satisfies a relational expression: $LM=\beta_1Z_1+\beta_2Z_2+\beta_3W+\beta_4H+\beta_5Age+\beta_6G+\beta_0$, where LM is the leg composition information, and $\beta_1$, $\beta_2$, $\beta_3$, $\beta_4$, $\beta_5$, $\beta_6$, and $\beta_0$ may be weighting coefficients determined experimentally.

The left leg composition information satisfies a relational expression: $LLM=\beta_{L1}Z_1+\beta_{L2}Z_2+\beta_{L3}W+\beta_{L4}H+\beta_{L5}Age+\beta_{L6}G+\beta_{L0}$, where LLM is the left leg composition information, and $\beta_{L1}$, $\beta_{L2}$, $\beta_{L3}$, $\beta_{L4}$, $\beta_{L3}$, $\beta_{L3}$, and $\beta_{L0}$ may be weighting coefficients determined experimentally. Further, left leg fat mass is $LLFM=\beta_{LF1}Z_1+\beta_{LF2}Z_2+\beta_{LF3}W+\beta_{LF4}H+\beta_{LF5}Age+\beta_{LF6}G+\beta_{LF0}$, and left leg muscle mass is $LLMM=P_{LM1}Z_1+P_{LM2}Z_2+P_{LM3}W+P_{LM4}H+P_{LM5}Age+P_{LM6}G+P_{LM0}$.

The right leg composition information satisfies a relational expression: $RLM=\beta_{R1}Z_1+\beta_{R2}Z_2+\beta_{R3}W+\beta_{R4}H+\beta_{R5}Age+\beta_{R6}G+\beta_{R0}$, where RLM is the right leg composition information, and $\beta_{R1}$, $\beta_{R2}$, $\beta_{R3}$, $\beta_{R4}$, $\beta_{R5}$, $\beta_{R6}$, and $\beta_{R0}$ may be weighting coefficients determined experimentally. Further, right leg fat mass is $RLFM=\beta_{RF1}Z_1+\beta_{RF2}Z_2+\beta_{RF3}W+\beta_{RF4}H+\beta_{RF5}Age+\beta_{RF6}G+\beta_{RF0}$, and right leg muscle mass is $RLMM=\beta_{RM1}Z_1+\beta_{RM2}Z_2+\beta_{RM3}W+\beta_{RM4}H+\beta_{RM5}Age+\beta_{RM6}G+\beta_{RM0}$.

Step 603: The four-electrode body fat scale determines second segmental body composition information of the detected person based on the weight information, the impedance information, and the first segmental body composition information of the detected person.

A second segment may be arms, and the arms include a left arm and a right arm.

In a first implementation, the four-electrode body fat scale determines arm composition information of the detected person based on the weight information, the impedance information, and the leg composition information of the detected person, where the arm composition information includes left arm composition information and right arm composition information.

In an example, the four-electrode body fat scale uses the weight information, the impedance information, the left leg composition information, and the right leg composition information of the detected person as input parameters, and inputs the input parameters into a trained third model. The model outputs the left arm composition information and the right arm composition information of the detected person. Model training may be performed in the four-electrode body fat scale, or may be performed in another device.

In another example, the four-electrode body fat scale determines the arm composition information of the detected person based on the weight information, a weighting coefficient of the weight information, the impedance information, a weighting coefficient of the impedance information, the leg composition information, and a weighting coefficient of the leg composition information.

For example, the arm composition information of the detected person satisfies a relational expression: $AM=\alpha_1Z_1+\alpha_2Z_2+\alpha_3W+\alpha_4LM+\alpha_0$, where AM (arm mass) is the arm composition information, LM is the leg composition information, $\alpha_1$, $\alpha_2$, $\alpha_3$, $\alpha_4$, and $\alpha_0$ may be weighting coefficients determined experimentally, $Z_1$ is the first impedance, $Z_2$ is the second impedance, and W is the weight information.

Specifically, the left arm composition information satisfies a relational expression: $LAM=\alpha_{L1}Z_1+\alpha_{L2}Z_2+\alpha_{L3}W+\alpha_{L4}LLM+\alpha_{L0}$, where LAM (left arm mass) is the left arm composition information, LLM is the left leg composition information, and $\alpha_{L1}$, $\alpha_{L2}$, $\alpha_{L3}$, $\alpha_{L4}$, and $\alpha_{L0}$ may be weighting coefficients determined experimentally. Further, left arm fat mass is $LAFM=\alpha_{LF1}Z_1+\alpha_{LF2}Z_2+\alpha_{LF3}W+\alpha_{LF4}LLFM+\alpha_{LF0}$, and left arm muscle mass is $LAMM=\alpha_{LM1}Z_1+\alpha_{LM2}Z_2+\alpha_{LM3}W+\alpha_{LM4}LLMM+\alpha_{LM0}$.

The right arm composition information satisfies a relational expression: $RAM=\alpha_{R1}Z_1+\alpha_{R2}Z_2+\alpha_{R3}W+\alpha_{R4}RLM+\alpha_{R0}$, where RAM (right arm mass) is the right arm composition information, RLM is the right leg composition information, and $\alpha_{R1}$,

$\alpha_{R2}$, $\alpha_{R3}$, $\alpha_{R4}$, and $\alpha_{R0}$ may be weighting coefficients determined experimentally. Further, right arm fat mass is $RAFM=\alpha_{RF1}Z_1+\alpha_{RF2}Z_2+\alpha_{RF3}W+\alpha_{RF4}RLFM+\alpha_{RF0}$, and right arm muscle mass is $RAMM=\alpha_{RM1}Z_1+\alpha_{RM2}Z_2+\alpha_{RM3}W+\alpha_{RM4}RLMM+\alpha_{RM0}$.

In addition, the four-electrode body fat scale may further determine the left arm composition information of the detected person with reference to the left leg composition information and the right leg composition information of the detected person, and determine the right arm composition information of the detected person with reference to the left leg composition information and the right leg composition information of the detected person.

Specifically, the left arm composition information satisfies a relational expression: $LAM=\alpha_{L1}Z_1+\alpha_{L2}Z_2+\alpha_{L3}W+\alpha_{L4}LLM+\alpha_{L5}RLM+\alpha_{L0}$, where LAM is the left arm composition information, LLM is the left leg composition information, RLM is the right leg composition information, and $\alpha_{L1}$, $\alpha_{L2}$, $\alpha_{L3}$, $\alpha_{L4}$, $\alpha_{L5}$, and $\alpha_{L0}$ may be weighting coefficients determined experimentally.

Further, the left arm fat mass is $LAFM=\alpha_{LF1}Z_1+\alpha_{LF2}Z_2+\alpha_{LF3}W+\alpha_{LF4}LLFM+\alpha_{LF5}RLFM+\alpha_{LF0}$, and the left arm muscle mass is $LAMM=\alpha_{LM1}Z_1+\alpha_{LM2}Z_2+\alpha_{LM3}W+\alpha_{LM4}LLMM+\alpha_{LM5}RLMM+\alpha_{LM0}$.

Furthermore, the four-electrode body fat scale may determine the left arm fat mass with reference to the left leg muscle mass and the right leg muscle mass. For example, the left arm fat mass is $LAFM=\alpha_{LF1}Z_1+\alpha_{LF2}Z_2+\alpha_{LF3}W+\alpha_{LF4}LLFM+\alpha_{LF5}RLFM+\alpha_{LF6}LLMM+\alpha_{LF7}RLMM+\alpha_{LF0}$.

The four-electrode body fat scale may further determine the left arm muscle mass with reference to the left leg fat mass and the right leg fat mass. For example, the left arm muscle mass is $LAMM=\alpha_{LM1}Z_1+\alpha_{LM2}Z_2+\alpha_{LM3}W+\alpha_{LM4}LLMM+\alpha_{LM5}RLMM+\alpha_{LM6}LLFM+\alpha_{LM7}RLFM+\alpha_{LM0}$.

When the four-electrode body fat scale determines the right arm composition information (the right arm fat mass and/or the right arm muscle mass), reference may be made to the implementation of determining the left arm composition information (the left arm fat mass and/or the left arm muscle mass) by the four-electrode body fat scale in the foregoing embodiment. Details are not described again.

To enable the four-electrode body fat scale to more accurately obtain the arm composition information of the detected person, this application provides a second implementation. The four-electrode body fat scale determines the arm composition information of the detected person based on the weight information, the impedance information, the left leg composition information, and the right leg composition information of the detected person and with reference to the first type of personal information of the detected person, for example, the gender, the age, the height, and the bust/waist/hip.

The four-electrode body fat scale determines the arm composition information by using the multivariate linear regression calculation method. The arm composition information satisfies a relational expression: $AM=\alpha_1Z_1+\alpha_2Z_2+\alpha_3W+\alpha_4H+\alpha_5Age+\alpha_6G+\alpha_7LM+\alpha_0$, where AM (arm mass) is the arm composition information, LM is the leg composition information, $\alpha_1$, $\alpha_2$, $\alpha_3$, $\alpha_4$, $\alpha_5$, $\alpha_6$, $\alpha_7$, and ao may be weighting coefficients determined experimentally, $Z_1$ is the first impedance, $Z_2$ is the second impedance, and W is the weight information.

Specifically, the left arm fat mass is LAFM=

$$\alpha_{LF1}Z_1+\alpha_{LF2}Z_2+\alpha_{LF3}W+\alpha_{LF4}LLFM+\alpha_{LF5}RLFM+\alpha_{LF8}H+\alpha_{LF9}Age+\alpha_{LF10}G+\alpha_{LF0}, \text{ or}$$

$$\alpha_{LF1}Z_1+\alpha_{LF2}Z_2+\alpha_{LF3}W+\alpha_{LF4}LLFM+\alpha_{LF5}RLFM+\alpha_{LF6}LLMM+\alpha_{LF7}RLMM+\alpha_{LF8}H+\alpha_{LF9}Age+\alpha_{LF10}G+\alpha_{LF0}.$$

The left arm muscle mass is LAMM=

$$\alpha_{LM1}Z_1+\alpha_{LM2}Z_2+\alpha_{LM3}W+\alpha_{LM4}LLMM+\alpha_{LM5}RLMM+\alpha_{LM8}H+\alpha_{LM9}Age+\alpha_{LM10}G+\alpha_{LM0}, \text{ or}$$

$$\alpha_{LM1}Z_1+\alpha_{LM2}Z_2+\alpha_{LM3}W+\alpha_{LM4}LLMM+\alpha_{LM5}RLMM+\alpha_{LM6}LLFM+\alpha_{LM7}RLFM+\alpha_{LM8}H+\alpha_{LM9}Age+\alpha_{LM10}G+\alpha_{LM0}.$$

The right arm fat mass is RAFM=

$$\alpha_{RF1}Z_1+\alpha_{RF2}Z_2+\alpha_{RF3}W+\alpha_{RF4}LLFM+\alpha_{RF5}RLFM+\alpha_{RF8}H+\alpha_{RF9}Age+\alpha\alpha_{RF10}G+\alpha_{RF0}, \text{ or}$$

$$\alpha_{RF1}Z_1+\alpha_{RF2}Z_2+\alpha_{RF3}W+\alpha_{RF4}LLFM+\alpha_{RF5}RLFM+\alpha_{RF6}LLMM+\alpha_{RF7}RLMM+\alpha_{RF8}H+\alpha_{RF9}Age+\alpha_{RF10}G+\alpha_{RF0}.$$

The right arm muscle mass is RAMM=

$$\alpha_{RM1}Z_1+\alpha_{RM1}Z_2+\alpha_{RM3}W+\alpha_{RM4}LLMM+\alpha_{RM5}RLMM+\alpha_{LM8}H+\alpha_{LM9}Age+\alpha_{LM10}G+\alpha_{RM0}, \text{ or}$$

$$\alpha_{RM1}Z_1+\alpha_{RM2}Z_2+\alpha_{RM3}W+\alpha_{RM4}LLMM+\alpha_{RM5}RLMM+\alpha_{RM6}LLFM+\alpha_{RM7}RLFM+\alpha_{RM8}H+\alpha_{RM9}Age+\alpha_{RM10}G+\alpha_{RM0}.$$

In addition, after step 603, the four-electrode body fat scale may further determine the whole-body composition information of the detected person.

In a first implementation, the four-electrode body fat scale determines the whole-body composition information of the detected person based on the weight information, the weighting coefficient of the weight information, the impedance information, the weighting coefficient of the impedance information, the leg composition information, and the weighting coefficient of the leg composition information.

Specifically, whole-body fat mass is $FM=\gamma_{F1}Z_1+\gamma_{F2}Z_2+\gamma_{F3}W+\gamma_{F4}LLFM+\gamma_{F5}RLFM+\gamma_{F8}H+\gamma_{F9}Age+\gamma_{F10}G+\gamma_{F0}$, or the whole-body fat mass is $FM=\gamma_{F1}Z_1+\gamma_{F2}Z_2+\gamma_{F3}W+\gamma_{F4}LLFM+\gamma_{F5}RLFM+\gamma_{F6}LLMM+\gamma_{F7}RLMM+\gamma_{F8}H+\gamma_{F9}Age+\gamma_{F10}G+\gamma_{F0}$, where $Z_1$ is the first impedance, $Z_2$ is the second impedance, W is the weight information, and $\gamma_{F1}$, ..., $\gamma_{F10}$, and $\gamma_{F0}$ may be weighting coefficients determined experimentally.

Specifically, whole-body muscle mass is $MM=\gamma_{M1}Z_1-\gamma_{M2}Z_2-\gamma_{M3}W+\gamma_{M4}LLMM-\gamma_{M5}RLMM-\gamma_{M5}H-\gamma_{M9}Age+\gamma_{M10}G-\gamma_{M0}$, or the whole-body muscle mass is $MM=\gamma_{M1}Z_1+\gamma_{M2}Z_2+\gamma_{M3}W+\gamma_{M4}LLMM+\gamma_{M5}RLMM+\gamma_{M6}LLFM+\gamma_{M7}RLFM+\gamma_{M8}H+\gamma_{M9}Age+\gamma_{M10}G+\gamma_{M0}$, where $Z_1$ is the first impedance, $Z_2$ is the second impedance, W is the weight information, and $\gamma_{M1}$, ..., $\gamma_{M10}$, and $\gamma_{M0}$ may be weighting coefficients determined experimentally.

In a second implementation, the four-electrode body fat scale determines the whole-body composition information of the detected person based on the weight information, the weighting coefficient of the weight information, the impedance information, the weighting coefficient of the impedance information.

Specifically, whole-body fat mass is $FM=\gamma_{F1}Z_1+\gamma_{F2}Z_2+\gamma_{F3}W+\gamma_{F8}H+\gamma_{F9}Age+\gamma_{F10}G+\gamma_{F0}$, where $Z_1$ is the first impedance, $Z_2$ is the second impedance, W is the weight information, and $\gamma_{F1}$, ..., $\gamma_{F10}$, and $\gamma_{F0}$ may be weighting coefficients determined experimentally. Whole-body muscle mass is $MM=\gamma_{M1}Z_1+\gamma_{M2}Z_2+\gamma_{M3}W+\gamma_{M8}H+\gamma_{M9}Age+\gamma_{M10}G+\gamma_{M0}$, where $Z_1$ is the first impedance, $Z_2$ is the second impedance, W is the weight information, and $\gamma_{M1}$, ..., $\gamma_{M10}$, and $\gamma_{M0}$ may be weighting coefficients determined experimentally.

In addition, after step 603, the four-electrode body fat scale may further determine third segmental body composition information of the detected person. A third segment may be a trunk, and the third segmental body composition information includes trunk fat mass and/or trunk muscle mass.

In a first implementation, the four-electrode body fat scale determines trunk composition information of the detected person based on the whole-body composition information, the leg composition information, and the arm composition information of the detected person. Specifically, the four-electrode body fat scale may subtract the leg composition information from the whole-body composition information of the detected person, and then subtract the arm composition information, to obtain the trunk composition information of the detected person. For example, the trunk fat mass is TrFM=FM-RLFM-LLFM-RAFM-LAFM, and the trunk muscle mass is TrMM=MM-RLMM-LLMM-RAMM-LAMM.

In a second implementation, the four-electrode body fat scale may determine trunk composition information of the detected person based on the weight information, the weighting coefficient of the weight information, the impedance information, the weighting coefficient of the impedance information, the leg composition information, and the weighting coefficient of the leg composition information. For details, reference may be made to the description of determining the arm composition information in step 603.

In addition, when the four-electrode body fat scale determines the whole-body composition information and partial-body composition information of the detected person, features $H^2/Z_1$ and $H^2/Z_2$ may be further added. With reference to $H^2/Z_1$, a weighting coefficient of $H^2/Z_1$, $H^2/Z_2$, and a weighting coefficient of $H^2/Z_2$, accuracy of determining human body compositions by the four-electrode body fat scale is further improved.

That the four-electrode body fat scale determines the whole-body muscle mass is used as an example. The whole-body muscle mass is $MM=\gamma_{M1}Z_1+\gamma_{M2}Z_2+\gamma_{M3}W+\gamma_{M8}H+\gamma_{M9}Age+\gamma_{M10}G+\gamma_{M11}H_t^2/Z_1+\gamma_{M12}H_t^2/Z_2$, where $Z_1$ is the first impedance, $Z_2$ is the second impedance, W is the weight information, and $\gamma_{M1}$, ..., $\gamma_{M12}$, and $\gamma_{M0}$ may be weighting coefficients determined experimentally.

In this application, when the four-electrode body fat scale determines the whole-body fat mass in the whole-body composition information of the detected person, or segmental body muscle mass or segmental body fat mass in segmental body composition information, reference may be made to the foregoing relational expressions. Details are not described again.

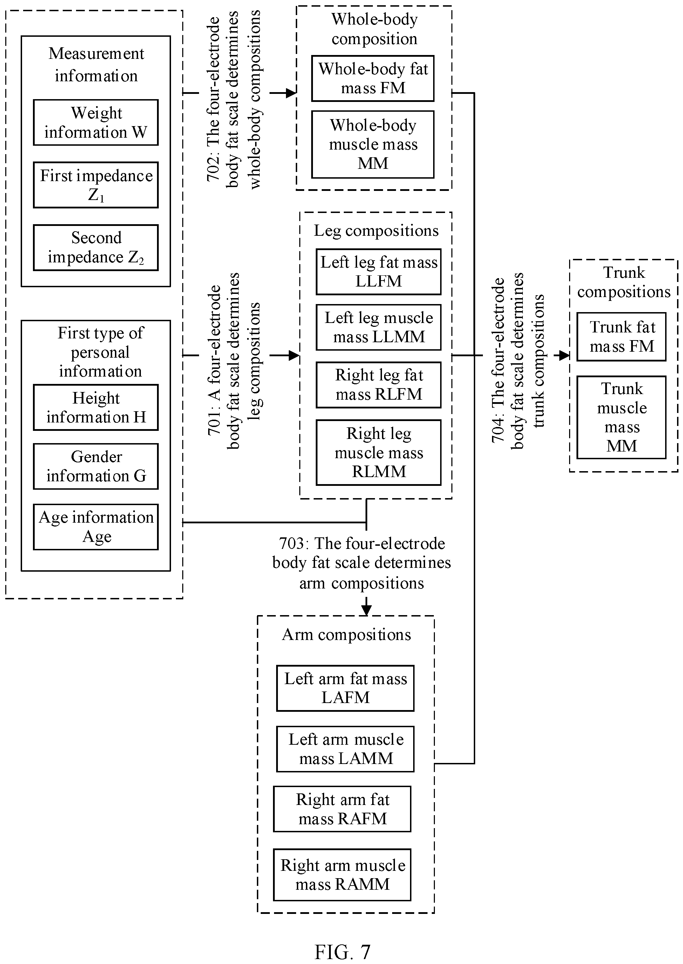

Based on the foregoing descriptions, FIG. 7 is a schematic flowchart of another human body composition measurement method according to this application. As shown in FIG. 7, measurement information of a detected person includes weight information W and impedance information (first impedance $Z_1$ and second impedance $Z_2$), and a first type of personal information includes height information H, gender information G, and age information Age.

Step 701: A four-electrode body fat scale determines leg compositions of the detected person based on the measurement information and the first type of personal information of the detected person. The leg compositions are, for example, left leg fat mass LLFM, right leg fat mass RLFM, left leg muscle mass LLMM, and right leg muscle mass RLMM.

Step 702: The four-electrode body fat scale determines whole-body compositions of the detected person based on the measurement information and the first type of personal information of the detected person. The whole-body compositions are, for example, whole-body fat mass FM and whole-body muscle mass MM.

Step 703: The four-electrode body fat scale determines arm compositions of the detected person based on the measurement information of the detected person, the first type of personal information, and the leg compositions of the detected person. The arm compositions are, for example, left arm fat mass LAFM, right arm fat mass RAFM, left arm muscle mass LAMM, and right arm muscle mass RAMM.

Step 704: The four-electrode body fat scale determines trunk compositions of the detected person based on the leg compositions, the whole-body compositions, and the arm compositions of the detected person. The trunk compositions are, for example, trunk muscle mass TrMM and trunk fat mass TrFM.

In this application, the four-electrode body fat scale may further determine human body composition information of the detected person with reference to a second type of personal information. The second type of personal information may be obtained by a band carried by the detected person, for example, motion information (for example, a current day's exercise amount and current exercise intensity), sleep information (for example, sleep quality and sleep duration), diet information, and heart information (for example, a heart rate) of the detected person. The second type of personal information may be used to determine segmental body composition information and the whole-body composition information in any step of step 601 to step 603 or in any step of step 701 to step 704, or may be used to correct the segmental body composition information and the whole-body composition information after step 603 or step 704.

In this embodiment of this application, after determining the whole-body compositions and the segmental body compositions of the detected person, the four-electrode body fat scale may further determine a limb balance indicator of the detected person based on the whole-body compositions and the segmental body compositions of the detected person, determine a balance result of the detected person based on the limb balance indicator of the detected person, and give a related exercise suggestion to the detected person.

Optionally, the limb balance indicator includes a balance indicator used to indicate muscle balance, for example, a first balance indicator, a second balance indicator, a third balance indicator, and a fourth balance indicator.

The first balance indicator is used to indicate a balance degree between left arm muscle and right arm muscle, the second balance indicator is used to indicate a balance degree between left leg muscle and right leg muscle, the third balance indicator is used to indicate a balance degree between arm muscle and whole-body muscle, and the fourth balance indicator is used to indicate a balance degree between leg muscle and the whole-body muscle.

For example, when determining the first balance indicator, the four-electrode body fat scale may specifically use a ratio of a first difference to first muscle mass as the first balance indicator. The first difference is a difference between the left arm muscle mass and the right arm muscle mass, and the first muscle mass is a sum of the left arm muscle mass and the right arm muscle mass. If the first balance indicator is greater than a first preset value, the four-electrode body fat scale determines that the arm muscle of the detected person is left/right unbalanced. If the first balance indicator is not greater than the first preset value, the four-electrode body fat scale determines that the arm muscle of the detected person is balanced.

For example, when determining the second balance indicator, the four-electrode body fat scale may specifically use a ratio of a second difference to second muscle mass as the second balance indicator. The second difference is a difference between the left leg muscle mass and the right leg muscle mass, and the second muscle mass is a sum of the left leg muscle mass and the right leg muscle mass. If the second balance indicator is greater than a second preset value, the four-electrode body fat scale determines that the leg muscle of the detected person is left/right unbalanced. If the second balance indicator is not greater than the second preset value, the four-electrode body fat scale determines that the leg muscle of the detected person is balanced.

For example, when determining the third balance indicator, the four-electrode body fat scale may specifically use a ratio of the first muscle mass to the whole-body muscle mass as the third balance indicator of the detected person. If the third balance indicator is less than a third preset value, the four-electrode body fat scale determines that a content of the arm muscle of the detected person is low. If the third balance indicator is not less than the third preset value, the four-electrode body fat scale determines that the arm muscle of the detected person is normal.

For example, when determining the fourth balance indicator, the four-electrode body fat scale may specifically use a ratio of the second muscle mass to the whole-body muscle mass as the fourth balance indicator of the detected person. If the fourth balance indicator is less than a fourth preset value, the four-electrode body fat scale determines that a content of the leg muscle of the detected person is low. If the fourth balance indicator is not less than the fourth preset value, the four-electrode body fat scale determines that the leg muscle of the detected person is normal.

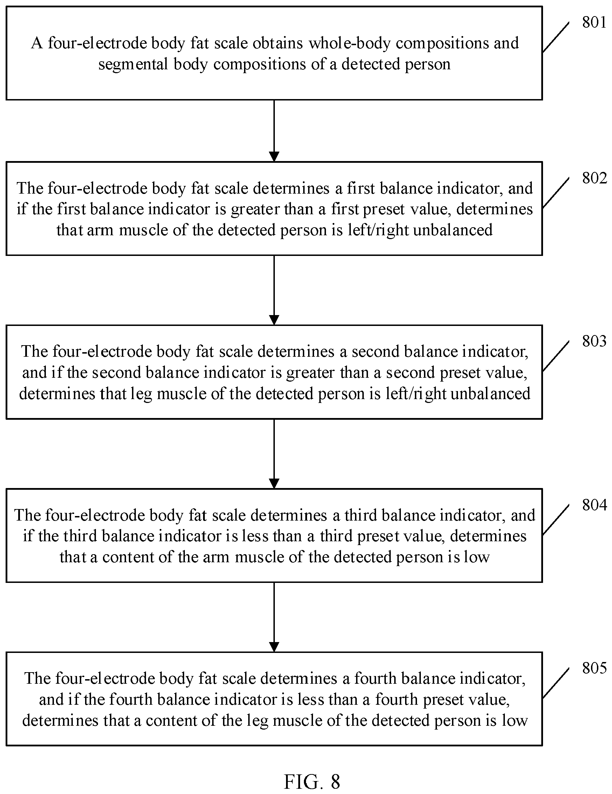

FIG. 8 is a schematic flowchart of determining a balance result of a detected person according to this application.

Step 801: A four-electrode body fat scale obtains whole-body compositions and segmental body compositions of a detected person.

Step 802: The four-electrode body fat scale determines a first balance indicator, and if the first balance indicator is greater than a first preset value, determines that arm muscle of the detected person is left/right unbalanced.

Step 803: The four-electrode body fat scale determines a second balance indicator, and if the second balance indicator is greater than a second preset value, determines that leg muscle of the detected person is left/right unbalanced.

Step 804: The four-electrode body fat scale determines a third balance indicator, and if the third balance indicator is less than a third preset value, determines that a content of the arm muscle of the detected person is low.

Step 805: The four-electrode body fat scale determines a fourth balance indicator, and if the fourth balance indicator is less than a fourth preset value, determines that a content of the leg muscle of the detected person is low.

In addition, the four-electrode body fat scale may further calculate balance indicators used to indicate fat balance. Calculation manners are similar to those described above, and details are not described again.

Further, the four-electrode body fat scale may determine the balance result of the detected person based on the first balance indicator, the second balance indicator, the third balance indicator, and the fourth balance indicator, and give a related exercise suggestion to the detected person.

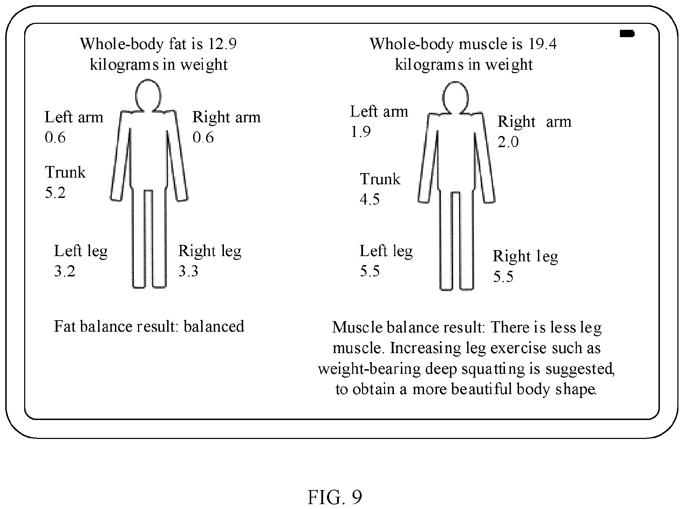

FIG. 9 is an example of a diagram of a display interface according to this application. The display interface may be a display interface of a four-electrode body fat scale. Specifically, after determining the foregoing information, the four-electrode body fat scale displays the foregoing information on the display interface of the four-electrode body fat scale. The display interface may be alternatively a display interface of the terminal device 220. After determining the foregoing information, the four-electrode body fat scale sends the foregoing information to the terminal device 220 by using the communication module 216, and the terminal device 220 displays the foregoing information on the display interface of the terminal device 220.

In the display interface shown in FIG. 9, whole-body fat of a detected person is 12.9 kilograms in weight, where left arm fat is 0.6 kilogram in weight, right arm fat is 0.6 kilogram in weight, trunk fat is 5.2 kilograms in weight, left leg fat is 3.2 kilograms in weight, and right leg fat is 3.3 kilograms in weight. A fat balance result is displayed below a fat indicator: balanced.

Whole-body muscle of the detected person is 19.4 kilograms in weight, where left arm muscle is 1.9 kilograms in weight, right arm muscle is 2.0 kilograms in weight, trunk muscle is 4.5 kilograms in weight, left leg muscle is 5.5 kilograms in weight, and right leg muscle is 5.5 kilograms in weight. A muscle balance result is displayed below a muscle indicator: There is less leg muscle. Increasing leg exercise such as weight-bearing deep squatting is suggested, to obtain a more beautiful body shape.

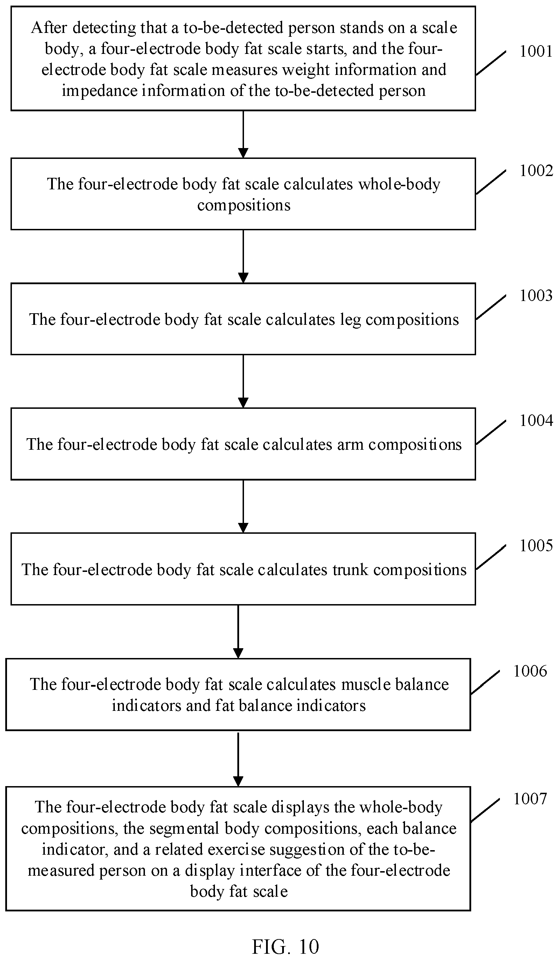

In addition, this application provides a detection procedure for determining segmental body compositions of a detected person by a four-electrode body fat scale. The procedure is specifically shown in FIG. 10.

Step 1001: After detecting that the detected person stands on a scale body, the four-electrode body fat scale starts, and the four-electrode body fat scale measures weight information and impedance information of the detected person.

Step 1002: The four-electrode body fat scale calculates whole-body compositions.

Step 1003: The four-electrode body fat scale calculates leg compositions.

Step 1004: The four-electrode body fat scale calculates arm compositions.

Step 1005: The four-electrode body fat scale calculates trunk compositions.

Step 1006: The four-electrode body fat scale calculates muscle balance indicators and fat balance indicators.

Step 1007: The four-electrode body fat scale displays the whole-body compositions, the segmental body compositions, each balance indicator, and a related exercise suggestion of the detected person on a display interface of the four-electrode body fat scale.

The foregoing has detailed a specific implementation in which the four-electrode body fat scale measures the weight information and the impedance information of the detected person and determines the body compositions of the detected person. The following provides an adjustment method, to improve accuracy of measuring weight information and impedance information of a detected person by a four-electrode body fat scale by adjusting two-foot contact information of the detected person on the four-electrode body fat scale. This is conducive to improving accuracy of determining body compositions of the detected person.

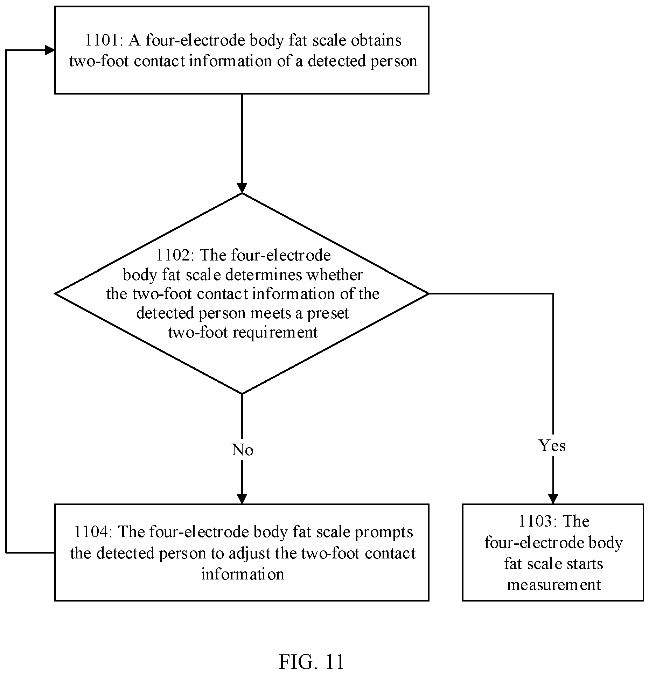

FIG. 11 is an example of a schematic flowchart of an adjustment method. The procedure is as follows.

Step 1101: A four-electrode body fat scale obtains two-foot contact information of a detected person.

The two-foot contact information includes standing posture information and/or standing location information. The standing posture information is, for example, two-foot spacing information and two-foot pressure information. The two-foot spacing information indicates that a two-foot spacing of the measurement object is greater than a distance threshold. The two-foot pressure information indicates that a pressure difference between two feet of the measurement object is not greater than a pressure difference threshold, that is, the two-foot pressure information is used to indicate whether the two feet stand in a balanced manner. The standing location information indicates that the two feet of the measurement object stand in a specified measurement region (which may also be referred to as a recommended region or a specified region) of the body fat measurement device. The specified measurement region includes the four electrodes shown in FIG. 4, so that related lower-body impedance can be measured accurately. For ease of description, the specified measurement region is referred to as a first specified region below.

For example, the four-electrode body fat scale may detect the two-foot spacing information, the two-foot pressure information, and the standing location information by using the pressure measurement module 211 in FIG. 3.

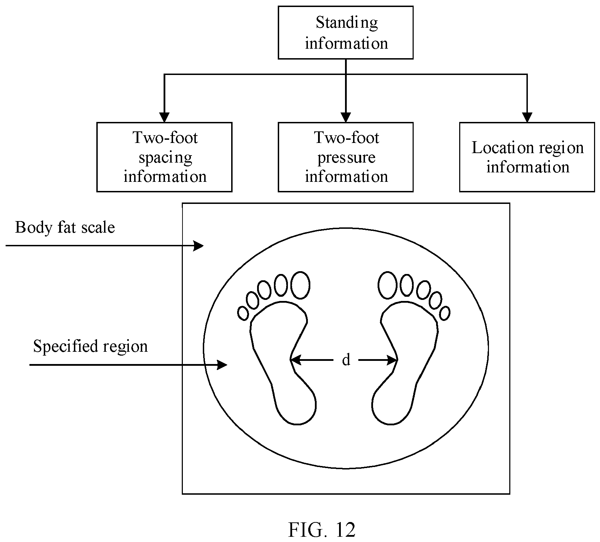

For example, FIG. 12 is a schematic diagram in which a four-electrode body fat scale detects two-foot contact information of a detected person when the detected person stands on the four-electrode body fat scale according to this application. For example, an ellipse in the figure is a first specified region of the four-electrode body fat scale, the detected person stands in the first specified region, and a distance between two feet is d. The four-electrode body fat scale can detect standing location information, two-foot spacing information, and two-foot pressure information of the detected person.

Step 1102: The four-electrode body fat scale determines whether the two-foot contact information of the detected person meets a preset two-foot requirement, and if the two-foot contact information meets the preset two-foot requirement, performs step 1103; otherwise, performs step 1104.

That the four-electrode body fat scale determines whether the two-foot contact information of the detected person meets a preset two-foot requirement includes: The four-electrode body fat scale determines whether the standing posture information of the detected person meets a first preset requirement, determines whether the standing location information of the detected person meets a second preset requirement, or determines whether the standing posture information of the detected person meets a first preset requirement and whether the standing location information of the detected person meets a second preset requirement.

The first preset requirement may include that the two feet are not in contact with each other (or two knees are not in contact with each other), and may be specifically that the two-foot spacing is greater than the distance threshold. The first preset requirement may further include that the two feet stand in a balanced manner, and may be specifically that the pressure difference between the two feet is not greater than the pressure difference threshold. The second preset requirement includes that the two feet of the detected person stand in the first specified region of the four-electrode body fat scale.

In other words, that the two-foot contact information meets the preset requirement includes at least one of the following: The two-foot spacing information indicates that the two-foot spacing of the measurement object is greater than the distance threshold; the two-foot pressure information indicates that the pressure difference between the two feet of the measurement object is not greater than the pressure difference threshold; and the standing location information indicates that the two feet of the measurement object stand in the first specified region of the body fat measurement device.

Step 1103: The four-electrode body fat scale starts measurement.

For a specific measurement manner, reference may be made to the embodiments related to FIG. 6, FIG. 7, FIG. 8, and FIG. 10. Details are not described herein again.

Step 1104: The four-electrode body fat scale prompts the detected person to adjust the two-foot contact information.

For example, if the standing posture information of the detected person does not meet the first preset requirement, the four-electrode body fat scale prompts the detected person to adjust a standing posture. If the standing location information of the detected person does not meet the second preset requirement, the four-electrode body fat scale prompts the detected person to adjust a standing location. The four-electrode body fat scale may prompt, by using a voice and/or interface display, the detected person to adjust the standing posture or the standing location.

With reference to the foregoing embodiment, this application provides a specific adjustment method. When the two feet of the detected person stand on the four-electrode body fat scale, the four-electrode body fat scale may sequentially verify whether the two feet of the detected person are in contact with each other, whether the two feet of the detected person stand in a balanced manner, and whether the detected person stands in the first specified region. In this application, a sequence of the three verification steps is not limited. After determining that verification in a previous step succeeds, the four-electrode body fat scale may perform verification in a next step. To be specific, alternatively, the four-electrode body fat scale may sequentially verify whether the two feet of the detected person stand in a balanced manner, whether the two feet of the detected person are in contact with each other, and whether the detected person stands in the first specified region, or perform verification in another sequence.

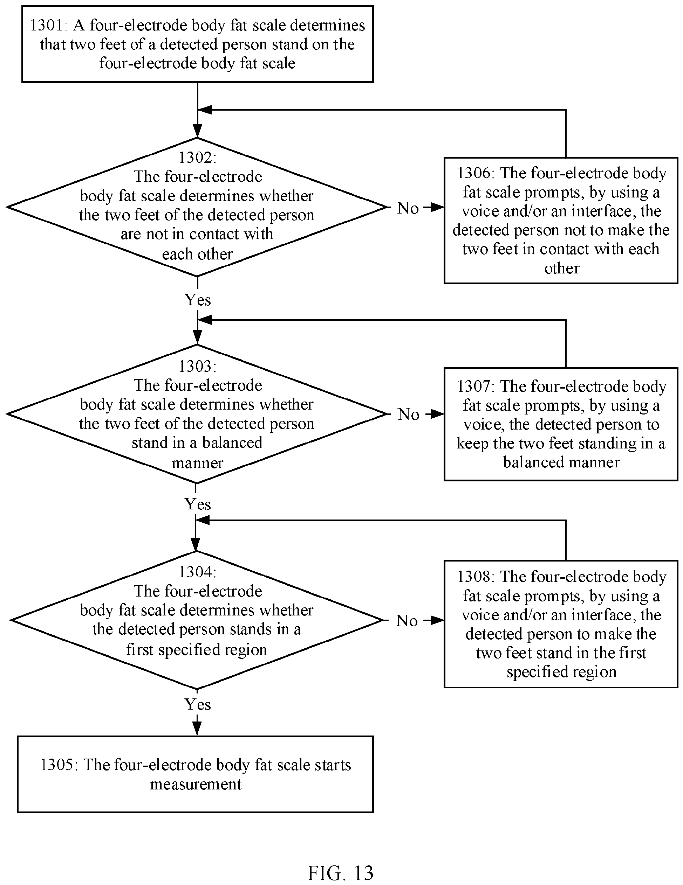

For example, a procedure shown in FIG. 13 is available.

Step 1301: The four-electrode body fat scale determines that the two feet of the detected person stand on the four-electrode body fat scale.

Step 1302: The four-electrode body fat scale determines whether the two feet of the detected person are not in contact with each other, and if the two feet of the detected person are not in contact with each other, performs step 1303; otherwise, performs step 1306. Specifically, the four-electrode body fat scale determines the two-foot spacing of the detected person. If the two-foot spacing is not greater than the distance threshold, it is determined that the two feet of the detected person are in contact with each other. If the two-foot spacing is greater than the distance threshold, it is determined that the two feet of the detected person are not in contact with each other. For example, a value of the distance threshold may be 0.

Step 1303: The four-electrode body fat scale determines whether the two feet of the detected person stand in a balanced manner, and if the two feet of the detected person stand in a balanced manner, performs step 1304; otherwise, performs step 1307. Specifically, the four-electrode body fat scale determines pressure on the two feet of the detected person. If the pressure difference between the two feet is greater than the pressure difference threshold, it is determined that the two feet of the detected person do not stand in a balanced manner. If the pressure difference between the two feet is not greater than the pressure difference threshold, it is determined that the two feet of the detected person stand in a balanced manner.

Step 1304: The four-electrode body fat scale determines whether the detected person stands in the first specified region, and if the detected person stands in the first specified region, performs step 1305; otherwise, performs step 1308. Specifically, by detecting a contact area between the two feet of the detected person and the four-electrode body fat scale, the four-electrode body fat scale determines locations of the two feet of the detected person, and determines whether the locations of the two feet of the detected person are in the first specified region.

Step 1305: The four-electrode body fat scale starts measurement, which means that the four-electrode body fat scale obtains the measurement information of the detected person.

For a specific measurement manner, reference may be made to the embodiments related to FIG. 6, FIG. 7, FIG. 8, and FIG. 10. Details are not described herein again.

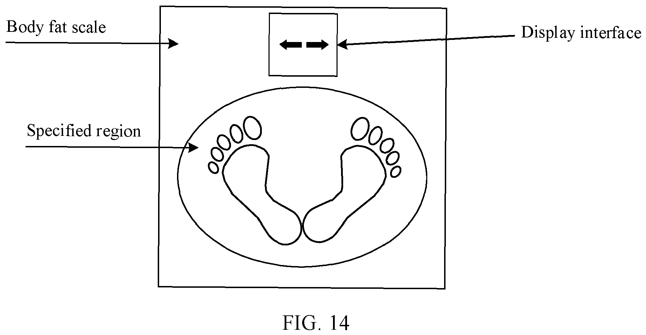

Step 1306: The four-electrode body fat scale prompts, by using a voice and/or an interface, the detected person not to make the two feet in contact with each other. The voice prompt is, for example, "Please stand with two feet apart". The interface prompt is, for example, displaying an animation on a display screen to guide the detected person to separate the two feet. For example, the display interface may be disposed on the four-electrode body fat scale. As shown in FIG. 14, rears of the two feet of the detected person are in contact with each other. The display interface is located above the four-electrode body fat scale. First prompt information is displayed on the display interface. The first prompt information includes a left arrow and a right arrow, and the two arrows jointly indicate the detected person to separate the two feet.

Step 1307: The four-electrode body fat scale prompts, by using a voice, the detected person to keep the two feet standing in a balanced manner. The voice prompt is, for example, "Please stand steadily".

Step 1308: The four-electrode body fat scale prompts, by using a voice and/or an interface, the detected person to make the two feet stand in the first specified region. The voice prompt is, for example, "Please stand in the middle of the four-electrode body fat scale".

The interface prompt is, for example, displaying an animation on the display screen to guide the detected person to stand in the first specified region. For example, the four-electrode body fat scale displays second prompt information on the display interface. The second prompt information includes an up arrow (indicating that the detected person moves forward), a down arrow (indicating that the detected person moves backward), a left arrow (indicating that the detected person moves left), a right arrow (indicating that the detected person moves right), a lower right arrow (indicating that the detected person moves backward to the right), an upper right arrow (indicating that the detected person moves forward to the right), a lower left arrow (indicating that the detected person moves backward to the left), and an upper left arrow (indicating that the detected person moves forward to the left).

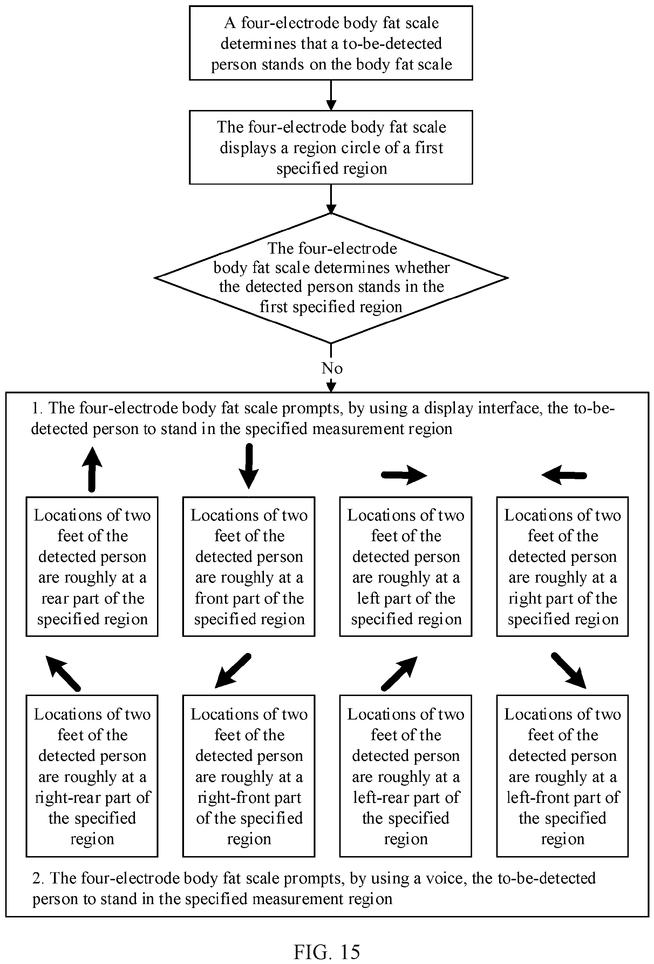

For example, FIG. 15 is an example of a schematic flowchart of determining, by a four-electrode body fat scale, whether a detected person stands in a first specified region according to this application. In a specific procedure, the four-electrode body fat scale displays a region circle of the first specified region, and the region circle indicates that the detected person stands in the first specified region. The four-electrode body fat scale determines whether the detected person stands in the first specified region; and if the detected person does not stand in the first specified region, prompts, based on an actual standing location of the detected person and the first specified region by using the second prompt information on the display interface, the detected person to adjust the standing location to the first specified region.

Specifically, if the locations of the two feet of the detected person are roughly at a rear part of the first specified region, the second prompt information is an up arrow. If the locations of the two feet of the detected person are roughly at a front part of the first specified region, the second prompt information is a down arrow. If the locations of the two feet of the detected person are roughly at a left part of the first specified region, the second prompt information is a right arrow. If the locations of the two feet of the detected person are roughly at a right part of the first specified region, the second prompt information is a left arrow. If the locations of the two feet of the detected person are roughly at a right-rear part of the first specified region, the second prompt information is an upper left arrow. If the locations of the two feet of the detected person are roughly at a right-front part of the first specified region, the second prompt information is a lower left arrow. If the locations of the two feet of the detected person are roughly at a left-rear part of the first specified region, the second prompt information is an upper right arrow. If the locations of the two feet of the detected person are roughly at a left-front part of the first specified region, the second prompt information is a lower right arrow.

In addition, the voice prompt "Please stand in the middle of the body fat scale" may be further provided.

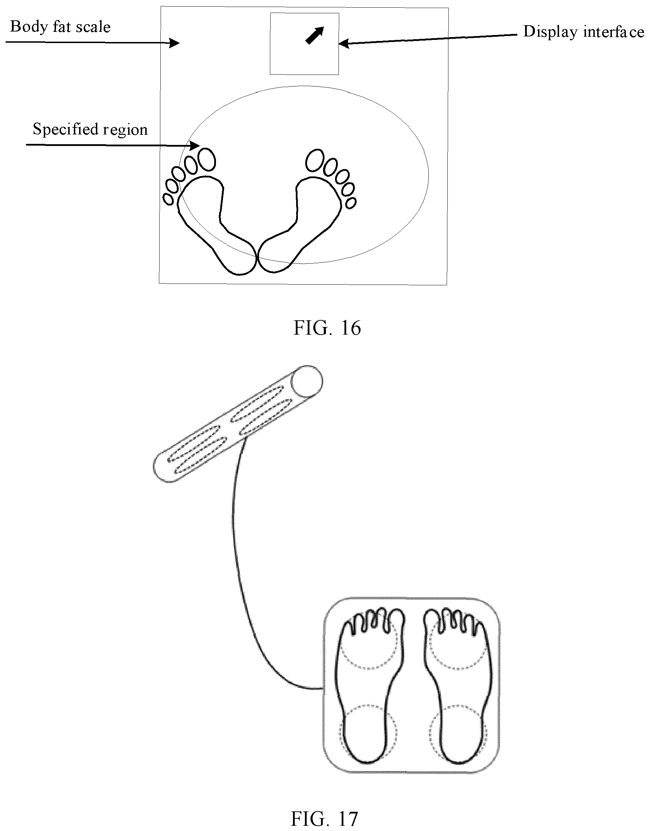

For example, FIG. 16 shows an example of a location at which two feet of a detected person stand on a four-electrode body fat scale. The second prompt information displayed on the display interface of the four-electrode body fat scale is an upper right arrow, indicating the detected person to move forward to the right.

It should be noted that, although the four-electrode body fat scale performs three verification procedures in the foregoing embodiment, in actual application, the four-electrode body fat scale may alternatively perform only one or two verification procedures, and start measurement after the verification ends.

In a second case, the body fat measurement device is an eight-electrode body fat scale.

A measurement principle of the eight-electrode body fat scale is first described as follows.

FIG. 17 is an example of a schematic diagram of an eight-electrode body fat scale according to this application. A handle is disposed on the eight-electrode body fat scale, and four electrodes are disposed in both a scale body and the handle of the eight-electrode body fat scale. For example, in FIG. 17, four dashed circles in the scale body represent four electrodes disposed in the scale body, and four dashed circles in the handle represent four electrodes disposed in the handle. Two feet of a detected person step on the scale body, front soles of the two feet are in contact with two electrodes in the scale body, and rear soles of the two feet are in contact with the other two electrodes in the scale body. In addition, two hands of the detected person hold the handle, front palms of the two hands are in contact with two electrodes in the handle, and rear palms of the two hands are in contact with the other two electrodes in the handle.

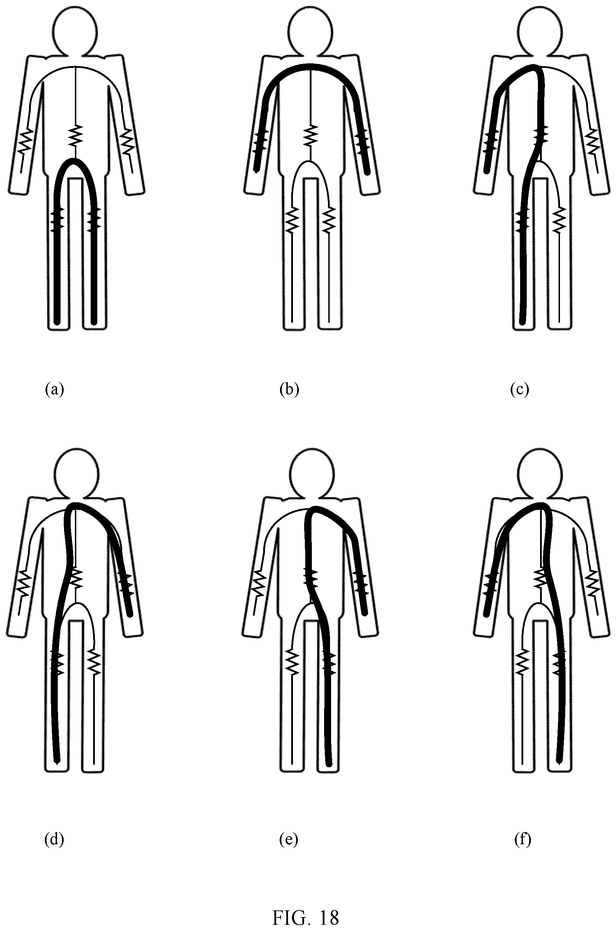

FIG. 18 is an example of a schematic diagram of a principle of detecting human body impedance by an eight-electrode body fat scale according to this application. The eight-electrode body fat scale may form six current loops in a human body: (a) is a current loop between two legs; (b) is a current loop between two arms; (c) is a current loop between a left upper limb, a trunk, and a left lower limb; (d) is a current loop between a right upper limb, the trunk, and the left lower limb; (e) is a current loop between the right upper limb, the trunk, and a right lower limb; and (f) is a current loop between the left upper limb, the trunk, and the right lower limb.

The eight-electrode body fat scale separately measures a voltage corresponding to the current loop between the two legs, a voltage corresponding to the current loop between the two arms, a voltage corresponding to the current loop between the left upper limb, the trunk, and the left lower limb, a voltage corresponding to the current loop between the right upper limb, the trunk, and the left lower limb, a voltage corresponding to the current loop between the right upper limb, the trunk, and the right lower limb, and a voltage corresponding to the current loop between the left upper limb, the trunk, and the right lower limb, and further calculates lower-body impedance, upper-body impedance, left-body impedance, right-upper left-lower body impedance, right-body impedance, and left-upper right-lower body impedance.

Then, the eight-electrode body fat scale determines left arm impedance, right arm impedance, left leg impedance, right leg impedance, and trunk impedance based on the lower-body impedance, the upper-body impedance, the left-body impedance, the right-upper left-lower body impedance, the right-body impedance, and the left-upper right-lower body impedance, and determines whole-body composition information and segmental body composition information based on the foregoing segment impedances.

It should be noted that, when the eight-electrode body fat scale is used, the handle may not be used, and only the four electrodes on the scale body are used. In this case, the eight-electrode body fat scale is equivalent to the four-electrode body fat scale, and can only measure the lower-body impedance. For a specific implementation, reference may be made to the human body composition measurement method performed by the four-electrode body fat scale in the first case.

However, the handle of the eight-electrode body fat scale may be incorrectly used. For example, because of a small contact area of an electrode of the handle, an incorrect arm posture of a user, an incorrect holding posture of the user, or a loose grasp of the user, the upper-body impedance, the left-body impedance, the right-upper left-lower body impedance, the right-body impedance, and the left-upper right-lower body impedance that are measured by the eight-electrode body fat scale are inaccurate. As a result, accuracy of the determined whole-body composition information and the determined segmental body composition information is affected.

Based on this, this application provides an adjustment method. The adjustment method may include the adjustment method based on the two-foot contact information and an adjustment method based on two-hand contact information. For the adjustment method based on the two-foot contact information, reference may be made to the implementation of the adjustment method based on the two-foot contact information in the four-electrode body fat scale in the embodiments related to FIG. 11 to FIG. 16. The following details the adjustment method based on the two-hand contact information.

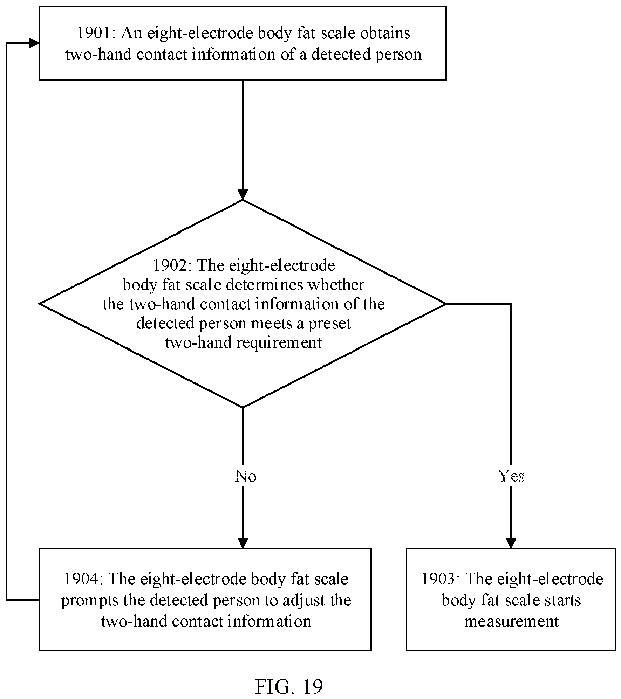

FIG. 19 is an example of a schematic flowchart of an adjustment method. The procedure is as follows.

Step 1901: An eight-electrode body fat scale obtains two-hand contact information of a detected person.

The two-hand contact information includes handle holding posture information and/or handle holding location information, where the handle holding posture information is, for example, handle holding area information and handle rope length information.

The handle holding area information may be a contact area between two hands and a handle. The handle holding area information indicates that a contact area between the two hands of the measurement object and the handle of the body fat measurement device is greater than an area threshold, that is, the handle holding area information is used to indicate whether a two-hand posture of the detected person is correct.

The handle rope length information may be a length of a rope that is pulled out of the handle connected to the rope when the handle in the eight-electrode body fat scale is pulled out from a scale body, and the handle rope length information is used to indicate whether a two-arm posture of the detected person is correct.

The handle holding location information is used to indicate whether the two hands of the detected person hold in a specified measurement region (which may also be referred to as a recommended region or a specified region) of the handle. The specified measurement region includes the four electrodes in the handle shown in FIG. 17, so that related upper-body impedance can be measured accurately. For ease of description, the specified measurement region is referred to as a second specified region below.

For example, the eight-electrode body fat scale may detect the handle holding area information, the handle rope length information, and the handle holding location information by using the pressure measurement module 211 in FIG. 3. The pressure measurement module 211 may be disposed in the handle and the scale body.

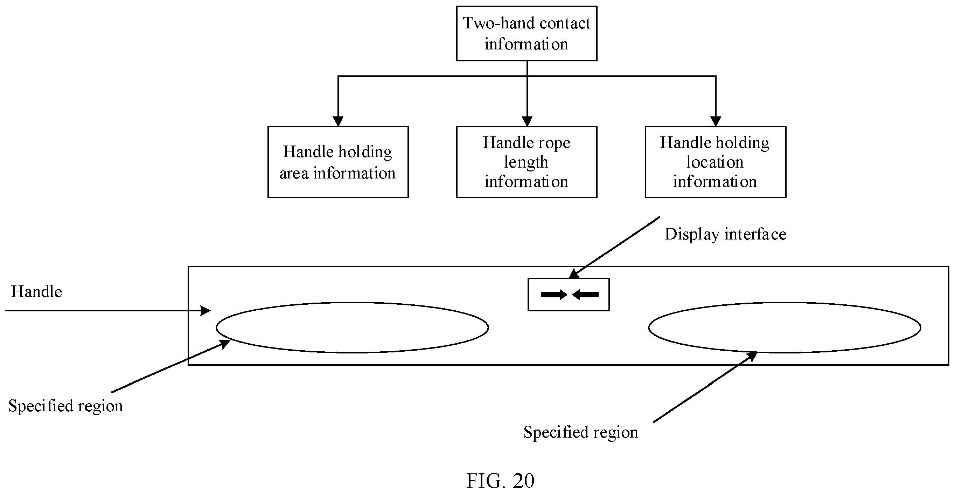

For example, FIG. 20 is a schematic diagram in which an eight-electrode body fat scale detects two-hand contact information of a detected person according to this application. For example, as shown in FIG. 20, two hands of the detected person hold in a second specified region, a handle of the eight-electrode body fat scale may detect handle holding area information, handle rope length information, and handle holding location information of the detected person.

Step 1902: The eight-electrode body fat scale determines whether the two-hand contact information of the detected person meets a preset two-hand requirement, and if the two-hand contact information meets the preset two-hand requirement, performs step 1903; otherwise, performs step 1904.

That the eight-electrode body fat scale determines whether the two-hand contact information of the detected person meets a preset two-hand requirement includes: The eight-electrode body fat scale determines whether the handle holding posture information of the detected person meets a third preset requirement, determines whether the handle holding location information of the detected person meets a fourth preset requirement, or determines whether the handle holding posture information of the detected person meets a third preset requirement and whether the handle holding location information of the detected person meets a fourth preset requirement.

The third preset requirement includes that the two-hand posture is correct, which may be specifically that the handle holding area information is greater than the area threshold. The third preset requirement further includes that the two-arm posture is correct, which may be specifically that two arms and a body fall within a preset angle range, and the handle rope length information falls within a preset rope length range. The fourth preset requirement includes that the two hands of the detected person hold in the second specified region.

In other words, that the two-hand contact information meets the preset requirement includes at least one of the following: The handle holding area information indicates that the contact area between the two hands of the measurement object and the handle of the body fat measurement device is greater than the area threshold; the handle rope length information indicates that a handle rope length of the body fat measurement device falls within the preset rope length range; and the handle holding location information indicates that the two hands of the measurement object hold in the second specified region of the handle of the body fat measurement device.

Step 1903: The eight-electrode body fat scale starts measurement.

For a specific measurement manner, reference may be made to the embodiment related to FIG. 17 and FIG. 18. Details are not described herein again.

Step 1904: The eight-electrode body fat scale prompts the detected person to adjust the two-hand contact information.

For example, if determining that the handle holding posture information of the detected person does not meet the third preset requirement, the eight-electrode body fat scale prompts the detected person to adjust a handle holding posture. If determining that the handle holding location information of the detected person does not meet the fourth preset requirement, the eight-electrode body fat scale prompts the detected person to adjust a handle holding location. The eight-electrode body fat scale may prompt, by using a voice and/or interface display, the detected person to adjust the handle holding posture or the handle holding location.

With reference to the foregoing embodiment, this application provides a specific adjustment method. The eight-electrode body fat scale may sequentially verify whether the two-hand posture of the detected person is correct, whether the two-arm posture of the detected person is correct, and whether the two hands of the detected person hold in the second specified region. In this application, a sequence of the three verification steps is not limited. After determining that verification in a previous step succeeds, the eight-electrode body fat scale may perform verification in a next step. To be specific, alternatively, the eight-electrode body fat scale may sequentially verify whether the two-arm posture of the detected person is correct, whether the two-hand posture of the detected person is correct, and whether the two hands of the detected person hold in the second specified region, or perform verification in another sequence.

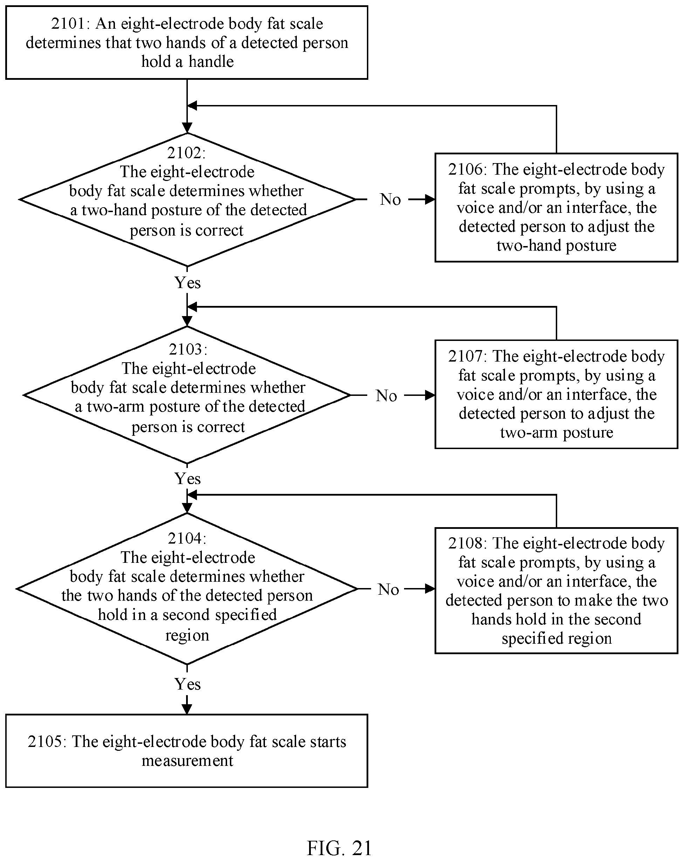

For example, a procedure shown in FIG. 21 is available.

Step 2101: The eight-electrode body fat scale determines that the two hands of the detected person hold the handle.

Step 2102: The eight-electrode body fat scale determines whether the two-hand posture of the detected person is correct, and if the two-hand posture of the detected person is correct, performs step 2103; otherwise, performs step 2106. Specifically, the eight-electrode body fat scale determines a contact area of the two hands of the detected person, and if determining that the contact area of the two hands is not greater than an area threshold, determines that the two-hand posture of the detected person is incorrect; or if determining that the contact area of the two hands is greater than the area threshold, determines that the two-hand posture of the detected person is correct.

Step 2103: The eight-electrode body fat scale determines whether the two-arm posture of the detected person is correct, and if the two-arm posture of the detected person is correct, performs step 2104; otherwise, performs step 2107. Specifically, the eight-electrode body fat scale determines the handle rope length information, and if the handle rope length information falls within the preset rope length range, determines that the two-arm posture of the detected person is correct; or if the handle rope length information does not fall within the preset rope length range, determines that the two-arm posture of the detected person is incorrect.

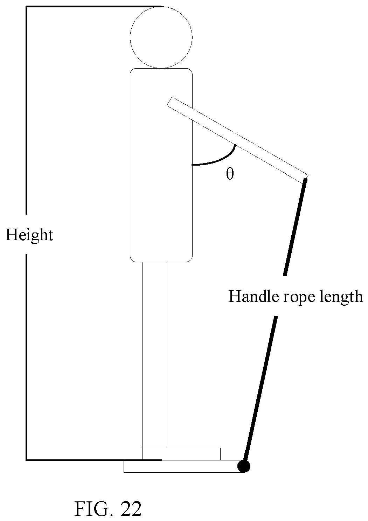

FIG. 22 is an example of a schematic diagram of determining, by an eight-electrode body fat scale, whether an arm of a detected person is far away from a body according to this application. A counter is disposed at a handle rope length of the eight-electrode body fat scale, and the counter is configured to measure a length of an extracted rope. The eight-electrode body fat scale may determine, based on a height of a user and the preset angle range, a rope length range of a rope extracted when the two-arm posture is correct, and then determine whether a length of an extracted rope falls within the length range. If the length of the extracted rope falls within the length range, the eight-electrode body fat scale determines that the two-arm posture of the detected person is correct; otherwise, determines that the two-arm posture of the detected person is incorrect.

Step 2104: The eight-electrode body fat scale determines whether the two hands of the detected person hold in the second specified region, and if the two hands of the detected person hold in the second specified region, performs step 2105; otherwise, performs step 2108.

Specifically, the second specified region includes a left-hand specified region and a right-hand specified region. For example, two ellipses in FIG. 20 are a left-hand specified region and a right-hand specified region. That the eight-electrode body fat scale determines whether the two hands of the detected person hold in the second specified region includes: The eight-electrode body fat scale determines whether a left hand of the detected person holds in the left-hand specified region, and determines whether a right hand of the detected person holds in the right-hand specified region. The eight-electrode body fat scale may determine, based on a contact region between the left hand and the handle, whether the left hand of the detected person holds in the left-hand specified region, and determine, based on a contact region between the right hand and the handle, whether the right hand of the detected person holds in the right-hand specified region.

Step 2105: The eight-electrode body fat scale starts measurement.

Step 2106: The eight-electrode body fat scale prompts, by using a voice and/or an interface, the detected person to adjust the two-hand posture. The voice prompt is, for example, "Please hold the handle with two hands correctly" or "Please hold the handle with two hands tightly".

Step 2107: The eight-electrode body fat scale prompts, by using a voice and/or an interface, the detected person to adjust the two-arm posture. The voice prompt is, for example, "Please keep two arms away from the body" or "Please adjust the arm angle".

Step 2108: The eight-electrode body fat scale prompts, by using a voice and/or an interface, the detected person to make the two hands hold in the second specified region. The voice prompt is, for example, "Please hold the correct location of the handle".

The interface prompt is, for example, displaying an animation on a display screen to guide the detected person to hold in the second specified region. For example, a display interface is disposed on the handle of the eight-electrode body fat scale, and third prompt information is displayed on the display interface. The third prompt information may include left-hand prompt information and right-hand prompt information. The left-hand prompt information includes a left arrow (indicating that the left hand of the detected person moves left) and a right arrow (indicating that the left hand of the detected person moves right), and the right-hand prompt information includes a left arrow (indicating that the right hand of the detected person moves left) and a right arrow (indicating that the right hand of the detected person moves right).

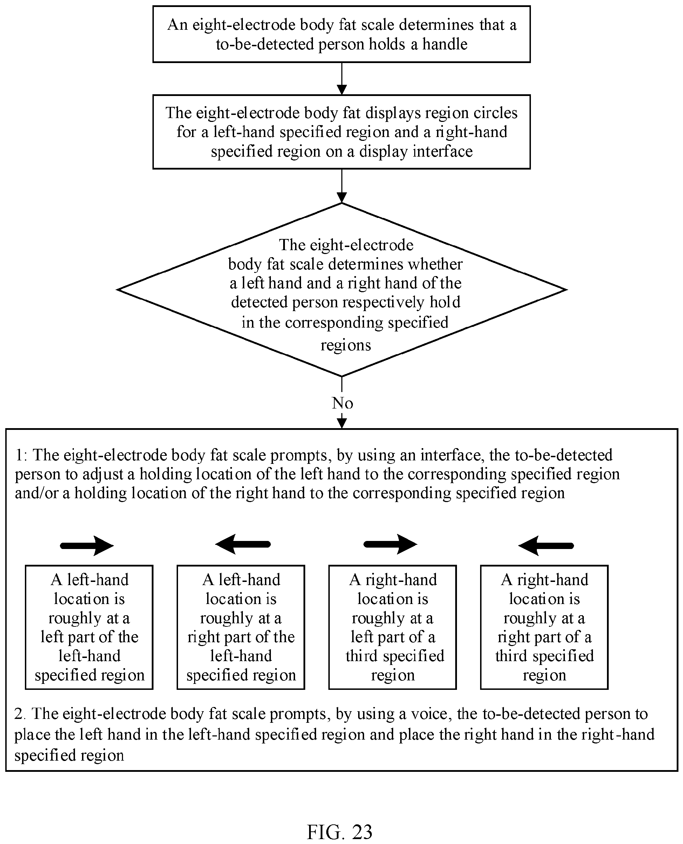

For example, FIG. 23 is an example of a schematic flowchart of determining, by an eight-electrode body fat scale, whether a detected person holds in a second specified region according to this application. In a specific procedure, the eight-electrode body fat scale determines that the detected person holds the handle, and displays region circles for the left-hand specified region and the right-hand specified region on the display interface of the handle. The region circle for the left-hand specified region indicates that the left hand of the detected person holds in the left-hand specified region, and the region circle for the right-hand specified region indicates that the right hand of the detected person holds in the right-hand specified region. The eight-electrode body fat scale determines whether the left hand and the right hand of the detected person respectively hold in the corresponding specified regions; and if the left hand and the right hand of the detected person do not respectively hold in the corresponding specified regions, prompts, based on an actual holding location of the detected person and the specified region by using the third prompt information on the display interface, the detected person to adjust a holding location of the left hand to the corresponding specified region and/or a holding location of the right hand to the corresponding specified region.

Specifically, if a left-hand location of the detected person is roughly at a left part of the left-hand specified region, the left-hand prompt information is a right arrow; or if a left-hand location of the detected person is roughly at a right part of the left-hand specified region, the left-hand prompt information is a left arrow; and

if a right-hand location of the detected person is roughly at a left part of the right-hand specified region, the right-hand prompt information is a right arrow; or if a right-hand location of the detected person is roughly at a right part of the right-hand specified region, the right-hand prompt information is a left arrow.

In addition, the eight-electrode body fat scale may further prompt the detected person by using a voice. The voice prompt is, for example, "Please hold the correct location of the handle", "Hold the left hand in the left region circle of the handle, and hold the right hand in the right region circle of the handle", or other voice prompt information.

It should be noted that, although the eight-electrode body fat scale performs three verification procedures in the foregoing embodiment, in actual application, the eight-electrode body fat scale may alternatively perform only one or two verification procedures, and start measurement after the verification ends.

The foregoing example shows the adjustment method based on the two-foot contact information and the adjustment method based on the two-hand contact information that are performed by the eight-electrode body fat scale. In this application, the eight-electrode body fat scale may further combine two-foot contact information and two-hand contact information to adjust a posture or a location of a detected person.

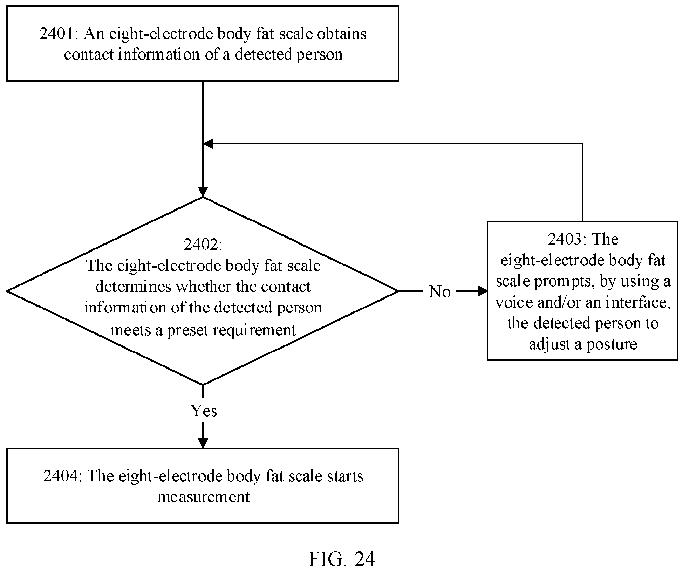

For details, reference may be made to a procedure in FIG. 24.

Step 2401: The eight-electrode body fat scale obtains contact information of the detected person, where the contact information includes the two-foot contact information and/or the two-hand contact information.

Step 2402: The eight-electrode body fat scale determines whether the contact information of the detected person meets a preset requirement, and if the contact information meets the preset requirement, performs step 2404; otherwise, performs step 2403. The eight-electrode body fat scale may specifically determine whether the two-foot contact information of the detected person meets a preset two-foot requirement, and/or determine whether the two-hand contact information of the detected person meets a preset two-hand requirement.

Step 2403: The eight-electrode body fat scale prompts, by using a voice and/or an interface, the detected person to adjust the posture. Step 2404: The eight-electrode body fat scale starts measurement.

A specific manner in this embodiment of this application has been described in other embodiments, and details are not described herein again.

In addition, in this application, the eight-electrode body fat scale may further determine human body composition information with reference to a second type of personal information. The second type of personal information may be obtained by a band carried by the detected person, for example, motion information (for example, a current day's exercise amount and current exercise intensity), sleep information (for example, sleep quality and sleep duration), diet information, and heart information (for example, a heart rate) of the detected person. The second type of personal information may be used in the foregoing embodiments related to measurement performed by the eight-electrode body fat scale, to correct the segmental body composition information and the whole-body composition information.

In this embodiment of this application, after determining the whole-body compositions and the segmental body compositions of the detected person, the eight-electrode body fat scale may further determine a limb balance indicator of the detected person based on the whole-body compositions and the segmental body compositions of the detected person, determine a balance result of the detected person based on the limb balance indicator of the detected person, and give a related exercise suggestion to the detected person. For details, reference may be made to the description of the first case.

In a third case, the body fat measurement device is a wearable device.

A measurement principle of the wearable device is first described as follows.

The wearable device includes four electrodes. In an example, two hands of a human body are in contact with the four electrodes at the same time. Specifically, a left hand of the human body is in contact with two electrodes, and a right hand of the human body is in contact with the other two electrodes, to form the current loop shown in (b) in FIG. 18, and obtain upper-body impedance of the human body.

In another example, one hand and one foot of a human body are in contact with four electrodes at the same time. Specifically, a left hand of the human body is in contact with two electrodes, and a left foot of the human body is in contact with the other two electrodes, to form the current loop shown in (c) in FIG. 18, and obtain left-body impedance of the human body;

a right hand of the human body is in contact with two electrodes, and a left foot of the human body is in contact with the other two electrodes, to form the current loop shown in (d) in FIG. 18, and obtain right-upper left-lower body impedance of the human body; a right hand of the human body is in contact with two electrodes, and a right foot of the human body is in contact with the other two electrodes, to form the current loop shown in (e) in FIG. 18, and obtain right-body impedance of the human body; or a left hand of the human body is in contact with two electrodes, and a right foot of the human body is in contact with the other two electrodes, to form the current loop shown in (f) in FIG. 18, and obtain left-upper right-lower body impedance of the human body.

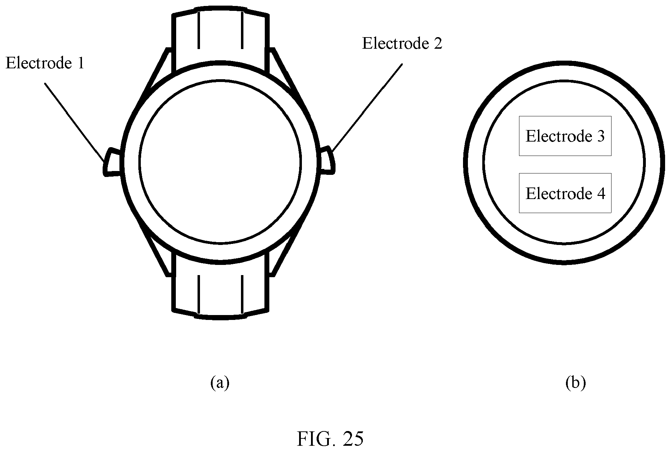

For example, the wearable device is a smart band. As shown in (a) in FIG. 25, sides of the smart band are provided with two electrodes: an electrode 1 and an electrode 2. As shown in (b) in FIG. 25, a location at which the smart band is in contact with human skin (a bottom of the smart band) is provided with the other two electrodes: an electrode 3 and an electrode 4.

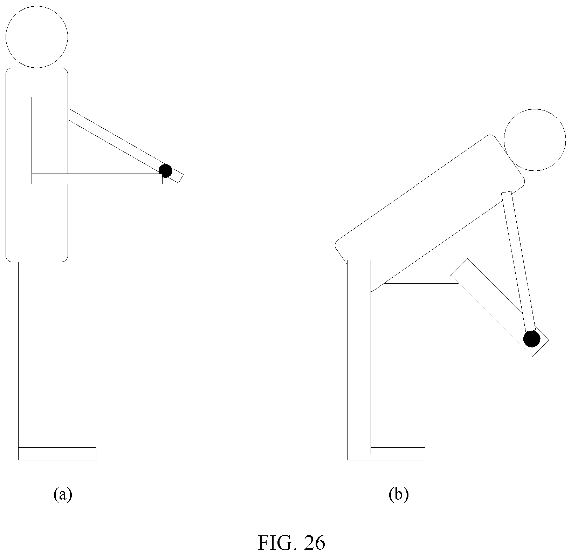

FIG. 26 is an example of a schematic diagram in which a detected person wears a wearable device according to this application. For example, in (a) in FIG. 26, the wearable device may be a smart band. When the detected person wears the smart band on a left hand, an electrode 3 and an electrode 4 of the smart band are in contact with a left wrist of the detected person, and fingers of a right hand of the detected person pinch an electrode 1 and an electrode 2, to form the current loop shown in (b) in FIG. 18. The smart band may measure upper-body impedance. In this example, a corresponding measurement manner may be referred to as a two-hand manner.

For another example, in (b) in FIG. 26, the wearable device may be a smart anklet. When the detected person wears the smart anklet on a left foot, an electrode 3 and an electrode 4 of the smart anklet are in contact with a left ankle of the detected person, and fingers of a right hand of the detected person pinch an electrode 1 and an electrode 2, to form the current loop shown in (d) in FIG. 18. The smart anklet may measure right-upper left-lower body impedance. In this example, a corresponding measurement manner may be referred to as a hand-foot manner.

FIG. 26 shows merely an example of a wearing manner according to this application. The detected person may further wear the wearable device at another location, for example, on a right foot. In this case, the electrode 3 and the electrode 4 of the wearable device are in contact with a right ankle of the detected person, and fingers of the left hand of the detected person pinch the electrode 1 and the electrode 2. The wearable device may measure left-upper right-lower body impedance. In this example, a corresponding measurement manner may be referred to as a hand-foot manner.

For example, when the measurement manner is a hand-foot manner, the measurement manner may include a first hand-foot manner, a second hand-foot manner, a third hand-foot manner, and a fourth hand-foot manner, which are respectively corresponding to four current loops (c) to (f) in FIG. 18, and are respectively corresponding to the left-body impedance, the right-upper left-lower body impedance, the right-body impedance, and the left-upper right-lower body impedance.

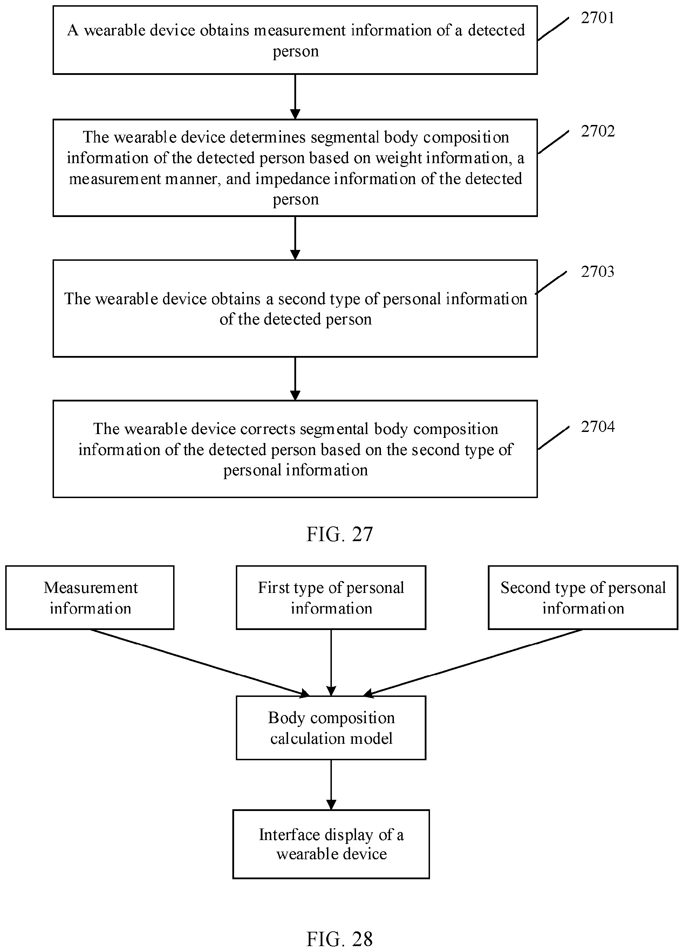

Based on the foregoing descriptions, this application provides a human body composition measurement method. For the method, reference may be made to a flowchart shown in FIG. 27.

Step 2701: The wearable device obtains measurement information of the detected person. The measurement information includes weight information, a measurement manner, and impedance information. The weight information of the detected person may be measured by a body fat scale. The weight information may be directly sent by the body fat scale to the wearable device, or forwarded by the body fat scale by using an intermediate device.

In an example, the wearable device is a smart band, and the intermediate device is a mobile phone of a user. The body fat scale sends the measured weight information to the mobile phone of the user, and then the smart band synchronizes user data with the mobile phone of the user. The user data includes the weight information of the detected person. In addition, the user data may further include a first type of personal information of the detected person, for example, an age, a height, a gender, and a weight.

The impedance information is obtained by the wearable device by measuring a first segment of the detected person, and the measurement manner is associated with the first segment. For example, the measurement manner is the first hand-foot manner, the first segment includes a left arm, a trunk, and a left leg, and the impedance information is the left-body impedance. For another example, the measurement manner is the second hand-foot manner, the first segment includes a right arm, the trunk, and the left leg, and the impedance information is the right-upper left-lower body impedance. For still another example, the measurement manner is the two-hand manner, the first segment includes the left arm and the right arm, and the impedance information is the upper-body impedance.

In this application, the measurement manner may be identified by a sensor module in the wearable device. Alternatively, the measurement manner may be identified based on historical measurement data and a difference between the measurement data.

Step 2702: The wearable device determines segmental body composition information of the detected person based on the weight information, the measurement manner, and the impedance information of the detected person.

Different measurement manners may be corresponding to different body composition calculation models. For example, the first hand-foot manner is corresponding to a body composition algorithm model 1, the second hand-foot manner is corresponding to a body composition algorithm model 2, the third hand-foot manner is corresponding to a body composition algorithm model 3, the fourth hand-foot manner is corresponding to a body composition algorithm model 4, and the two-hand manner is corresponding to a body composition algorithm model 5.

In a first example, if the measurement manner is the first hand-foot manner, the wearable device may determine the segmental body composition information of the detected person based on the weight information, the impedance information, the first type of personal information, and the body composition algorithm model 1 of the detected person. If the measurement manner is the two-hand manner, the wearable device may determine the segmental body composition information of the detected person based on the weight information, the impedance information, the first type of personal information, and the body composition algorithm model 5 of the detected person. Other measurement manners are similar to those described above, and details are not described again.

In a second example, if the measurement manner is the first hand-foot manner, the wearable device may first determine first segmental body composition information based on the weight information, the impedance information, and the first type of personal information of the detected person, and then input the weight information, the impedance information, and the first segmental body composition information of the measurement object into the body composition algorithm model 1, to obtain other segmental body composition information of the detected person. If the measurement manner is the two-hand manner, the wearable device may first determine first segmental body composition information based on the weight information, the impedance information, and the first type of personal information of the detected person, and then input the weight information, the impedance information, and the first segmental body composition information of the measurement object into the body composition algorithm model 5, to obtain other segmental body composition information of the detected person.

Step 2703: The wearable device obtains a second type of personal information of the detected person. The second type of personal information is, for example, current motion information (for example, a current day's exercise amount and exercise intensity), sleep information (for example, sleep quality and sleep duration), diet information, and heart information (for example, a heart rate) of the detected person. The second type of personal information may be obtained by the wearable device by continuously monitoring the detected person. For example, the wearable device is a smart band, the detected person continuously wears the smart band, and the smart band may obtain the second type of personal information of the detected person.

Step 2704: The wearable device corrects the segmental body composition information of the detected person based on the second type of personal information.

The wearable device corrects the segmental body compositions of the detected person based on the current day's exercise amount, the exercise intensity, the sleep information, the heart information, the diet information, and the like of the detected person, to obtain the corrected segmental body compositions of the detected person.

Optionally, the segmental body composition information of the detected person and/or a limb balance indicator of the detected person may be displayed on a display interface of the wearable device. For details of this embodiment, reference may be made to the embodiments related to FIG. 8, FIG. 9, and FIG. 10.

In this implementation, the wearable device may determine the segmental body composition information and/or the limb balance indicator of the detected person, set a personalized fitness solution during exercise fitness based on a corresponding intelligent suggestion, evaluate a current exercise effect based on exercise detail information, consumed calories, and the like that are recorded by the wearable device, make a diet suggestion based on results such as a basal metabolic rate and a body mass index (body mass index, BMI), and perform other operations.

For example, with reference to FIG. 28, the wearable device may input the measurement information, the first type of personal information, and the second type of personal information into a corresponding body composition calculation model to obtain the segmental body composition information and the limb balance indicator of the detected person, and display the segmental body composition information and the limb balance indicator on the display interface of the wearable device.

In this application, the wearable device is used to determine the body composition information of the detected person, and measurement is convenient. In addition, the wearable device may be usually associated with a single user, and can continuously monitor a second type of personal information of the user, to obtain accurate human body composition information. Further, the wearable device may provide human body health status evaluation, personalized life and fitness suggestions, and the like for the user based on the current human body composition information. This is conducive to implementing desirable user experience.

Embodiments described in this specification may be independent solutions, or may be combined based on internal logic. All these solutions fall within the protection scope of this application.

In the foregoing embodiments provided in this application, the methods provided in embodiments of this application are described from a perspective of interaction between devices. To implement the functions in the methods provided in the foregoing embodiments of this application, the body fat measurement device may include a hardware structure and/or a software module, to implement the foregoing functions by using the hardware structure, the software module, or a combination of the hardware structure and the software module. Whether a function in the foregoing functions is performed by using a hardware structure, a software module, or a combination of a hardware structure and a software module depends on particular applications and design constraints of the technical solutions.

Module division in embodiments of this application is an example, is merely logical function division, and may be other division in actual implementation. In addition, functional modules in embodiments of this application may be integrated into one processor, or may exist alone physically, or two or more modules may be integrated into one module. The integrated module may be implemented in a form of hardware, or may be implemented in a form of a software functional module.

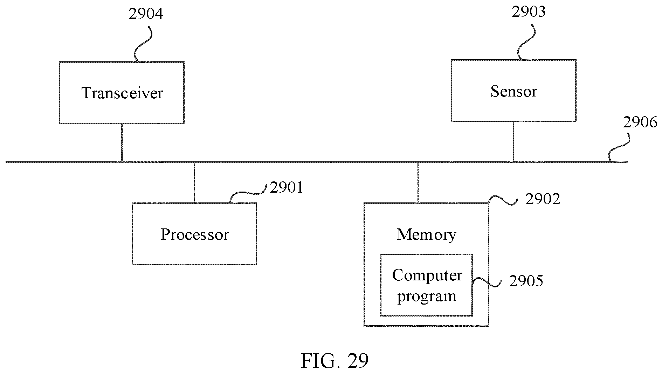

Based on the foregoing content and a same concept, FIG. 29 is a possible schematic diagram of a structure of a body fat measurement device according to this application. The body fat measurement device may be the body fat measurement device in the foregoing method embodiments, may be configured to implement functions of the body fat measurement device in the foregoing method embodiments, and therefore can also implement the beneficial effects of the foregoing method embodiments.

In this application, the body fat measurement device may be the body fat measurement device 210 shown in FIG. 2, or may be a module (for example, a chip) applied to the body fat measurement device.

As shown in FIG. 29, the body fat measurement device includes one or more processors 2901, a memory 2902, a sensor 2903, a transceiver 2904, and one or more computer programs 2905. The components may be connected through one or more communication buses 2906. Although not shown, the body fat measurement device may further include more components such as a display and a loudspeaker.

The one or more computer programs 2905 are stored in the memory 2902, the one or more computer programs 2905 include instructions, and when the instructions are invoked and executed by the one or more processors 2901, the body fat measurement device is enabled to perform the following steps: controlling the sensor 2903 to obtain impedance information of a measurement object by measuring a first segment of the measurement object; controlling the sensor 2903 or the transceiver 2904 to obtain weight information of the measurement object; determining first segmental body composition information of the measurement object based on the weight information and the impedance information; and determining second segmental body composition information of the measurement object based on the weight information, the impedance information, and the first segmental body composition information of the measurement object.

In a possible implementation, the impedance information includes first impedance and second impedance, the first impedance is obtained by the body fat measurement device by controlling the sensor 2903 to measure the first segment by using a measurement signal at a first frequency, the second impedance is obtained by the body fat measurement device by controlling the sensor 2903 to measure the first segment by using a measurement signal at a second frequency, and the first frequency is less than the second frequency.

In a possible implementation, the first frequency is 50 kHz, and the second frequency is 250 kHz.

In a possible implementation, the second segmental body composition information of the measurement object satisfies the following relational expression:

$$AM = \alpha_1 Z_1 + \alpha_2 Z_2 + \alpha_3 W + \alpha_4 H + \alpha_5 Age + \alpha_6 G + \alpha_7 LM + \alpha_0,$$

where
AM is the second segmental body composition information, $Z_1$ is the first impedance, $\alpha_1$ is a weighting coefficient of the first impedance, $Z_2$ is the second impedance, $\alpha_2$ is a weighting coefficient of the second impedance, W is the weight information of the measurement object obtained by the body fat measurement device by controlling the sensor 2903 or the transceiver 2904, $\alpha_3$ is a weighting coefficient of the weight information, H is height information of the measurement object obtained by the body fat measurement device by controlling the transceiver 2904, $\alpha_4$ is a weighting coefficient of the height information, Age is age information of the measurement object obtained by the body fat measurement device by controlling the transceiver 2904, $\alpha_5$ is a weighting coefficient of the age information, G is gender information of the measurement object obtained by the body fat measurement device by controlling the transceiver 2904, a value of G is 1 or 0, $\alpha_6$ is a weighting coefficient of the gender information, LM is the first segmental body composition information, $\alpha_7$ is a weighting coefficient of the first segmental body composition information, and $\alpha_0$ is a constant.

In a possible implementation, when the instructions are invoked and executed by the one or more processors 2901, the body fat measurement device is further enabled to perform the following steps: determining whole-body composition information of the measurement object based on the weight information, a weighting coefficient of the weight information, the impedance information, and a weighting coefficient of the impedance information; and determining third segmental body composition information of the measurement object based on the whole-body composition information, the first segmental body composition information, and the second segmental body composition information of the measurement object.

In a possible implementation, the body fat measurement device is a four-electrode body fat scale, the first segmental body composition information includes left leg composition information and/or right leg composition information, the second segmental body composition information includes left arm composition information and/or right arm composition information, and the third segmental body composition information includes trunk composition information.

In a possible implementation, when the instructions are invoked and executed by the one or more processors 2901, the body fat measurement device is further enabled to perform the following step: determining a limb balance indicator of the measurement object based on the whole-body composition information, the first segmental body composition information, the second segmental body composition information, and the third segmental body composition information.

In a possible implementation, the body fat measurement device further includes a display; and when the instructions are invoked and executed by the one or more processors 2901, the body fat measurement device is further enabled to perform the following step: controlling the display to display any one or more of the following: the first segmental body composition information, the second segmental body composition information, the third segmental body composition information, the whole-body composition information, the limb balance indicator, and an exercise suggestion corresponding to the limb balance indicator of the measurement object.

In a possible implementation, the body fat measurement device is a wearable device, the measurement information further includes a measurement manner, and the measurement manner is corresponding to the first segment; and when the instructions are invoked and executed by the one or more processors 2901, the body fat measurement device is further enabled to perform the following step: inputting the weight information, the impedance information, and the first segmental body composition information of the measurement object into a body composition algorithm model corresponding to the measurement manner, to obtain the second segmental body composition information of the measurement object.

In a possible implementation, when the instructions are invoked and executed by the one or more processors 2901, the body fat measurement device is further enabled to perform the following steps: controlling the sensor 2903 to obtain contact information between the measurement object and the body fat measurement device, where the contact information includes two-hand contact information and/or two-foot contact information between the body fat measurement device and the measurement object; and determining that the contact information of the measurement object meets a preset requirement.

In a possible implementation, the two-foot contact information includes two-foot spacing information, two-foot pressure information, and standing location information; and that the two-foot contact information meets a preset requirement includes at least one of the following: The two-foot spacing information indicates that a two-foot spacing of the measurement object is greater than a distance threshold; the two-foot pressure information indicates that a pressure difference between two feet of the measurement object is not greater than a pressure difference threshold; and the standing location information indicates that the two feet of the measurement object stand in a first specified region of the body fat measurement device.

In a possible implementation, the two-hand contact information includes handle holding area information, handle rope length information, and handle holding location information; and that the two-hand contact information meets a preset requirement includes at least one of the following: The handle holding area information indicates that a contact area between two hands of the measurement object and a handle of the body fat measurement device is greater than an area threshold; the handle rope length information indicates that a handle rope length of the body fat measurement device falls within a preset rope length range; and the handle holding location information indicates that the two hands of the measurement object hold in a second specified region of the handle of the body fat measurement device.

In a possible implementation, the body fat measurement device is a four-electrode body fat scale, and the contact information includes the two-foot contact information; or the body fat measurement device is an eight-electrode body fat scale, and the contact information includes the two-hand contact information and the two-foot contact information.

Based on the foregoing content and a same concept, a computer-readable storage medium is provided. The computer-readable storage medium stores a computer program or instructions, and when the computer program or instructions are executed by a computer, any method in the method embodiments shown in FIG. 6 to FIG. 28 is implemented.

Based on the foregoing content and a same concept, this application provides a computer program product. The computer program product includes a computer program or instructions, and when the computer program or the instructions are executed by a computer, any method in the method embodiments shown in FIG. 6 to FIG. 28 is implemented.

A person skilled in the art should understand that embodiments of this application may be provided as methods, systems, or a computer program product. Therefore, this application may use a form of hardware only embodiments, software only embodiments, or embodiments with a combination of software and hardware. Moreover, this application may use a form of a computer program product that is implemented on one or more computer-usable storage media (including but not limited to a disk memory, an optical memory, and the like) that include computer usable program code.

This application is described with reference to the flowcharts and/or block diagrams of the methods, the devices (systems), and the computer program product according to this application. It should be understood that computer program instructions may be used to implement each process in the flowcharts and/or each block in the block diagrams and a combination of a process in the flowcharts and/or a block in the block diagrams. These computer program instructions may be provided for a general-purpose computer, a dedicated computer, an embedded processor, or a processor of another programmable data processing device to generate a machine, so that the instructions executed by the computer or the processor of the another programmable data processing device generate an apparatus for implementing a specific function in one or more processes in the flowcharts and/or in one or more blocks in the block diagrams.

These computer program instructions may be stored in a computer-readable memory that can instruct the computer or the another programmable data processing device to work in a specific manner, so that the instructions stored in the computer-readable memory generate an artifact that includes an instruction apparatus. The instruction apparatus implements a specific function in one or more processes in the flowcharts and/or in one or more blocks in the block diagrams.

Obviously, a person skilled in the art can make various modifications and variations to this application without departing from the scope of this application. This application is intended to cover these modifications and variations of this application provided that they fall within the scope defined by the following claims and their equivalent technologies of this application.

**Claims**

1. A human body composition measurement method, comprising:

   obtaining, by a body fat measurement device, measurement information of a measurement object, wherein the measurement information comprises weight information and impedance information, and the impedance information is obtained by measuring a first segment of the measurement object;
   determining, by the body fat measurement device, first segmental body composition information of the measurement object based on the weight information and the impedance information; and
   determining, by the body fat measurement device, second segmental body composition information of the measurement object based on the weight information, the impedance information, and the first segmental body composition information of the measurement object.

2. The method according to claim 1, wherein the impedance information comprises first impedance and second impedance, the first impedance is obtained by measuring the first segment by using a measurement signal at a first frequency, the second impedance is obtained by measuring the first segment by using a measurement signal at a second frequency, and the first frequency is less than the second frequency.

3. The method according to claim 2, wherein the first frequency is 50 kHz, and the second frequency is 250 kHz.

4. The method according to claim 2 or 3, wherein the second segmental body composition information of the measurement object satisfies the following relational expression:

$$AM = \alpha_1 Z_1 + \alpha_2 Z_2 + \alpha_3 W + \alpha_4 H + \alpha_5 Age + \alpha_6 G + \alpha_7 LM + \alpha_0,$$

   wherein
   AM is the second segmental body composition information, $Z_1$ is the first impedance, $\alpha_1$ is a weighting coefficient of the first impedance, $Z_2$ is the second impedance, $\alpha_2$ is a weighting coefficient of the second impedance, W is the weight information of the measurement object obtained by the body fat measurement device, $\alpha_3$ is a weighting coefficient of the weight information, H is height information of the measurement object obtained by the body fat measurement device, $\alpha_4$ is a weighting coefficient of the height information, Age is age information of the measurement object obtained by the body fat measurement device, $\alpha_5$ is a weighting coefficient of the age information, G is gender information of the measurement object obtained by the body fat measurement device, a value of G is 1 or 0, $\alpha_6$ is a weighting coefficient of the gender information, LM is the first segmental body composition information, $\alpha_7$ is a weighting coefficient of the first segmental body composition information, and ao is a constant.

5. The method according to claim 1, wherein the method further comprises:

   determining, by the body fat measurement device, whole-body composition information of the measurement object based on the weight information, a weighting coefficient of the weight information, the impedance information, and a weighting coefficient of the impedance information; and
   determining, by the body fat measurement device, third segmental body composition information of the measurement object based on the whole-body composition information, the first segmental body composition information, and the second segmental body composition information of the measurement object.

6. The method according to claim 5, wherein the body fat measurement device is a four-electrode body fat scale, the first segmental body composition information comprises left leg composition information and/or right leg composition information, the second segmental body composition information comprises left arm composition information and/or right arm composition information, and the third segmental body composition information comprises trunk composition information.

7. The method according to claim 5 or 6, wherein the method further comprises:
determining a limb balance indicator of the measurement object based on the whole-body composition information, the first segmental body composition information, the second segmental body composition information, and the third segmental body composition information.

8. The method according to claim 7, wherein the method further comprises:
displaying, by the body fat measurement device, any one or more of the following on a display interface of the body fat measurement device: the first segmental body composition information, the second segmental body composition information, the third segmental body composition information, the whole-body composition information, the limb balance indicator, and an exercise suggestion corresponding to the limb balance indicator of the measurement object.

9. The method according to claim 1, wherein the body fat measurement device is a wearable device, the measurement information further comprises a measurement manner, and the measurement manner is corresponding to the first segment; and
the determining, by the body fat measurement device, second segmental body composition information of the measurement object based on the weight information, the impedance information, and the first segmental body composition information of the measurement object comprises:
inputting, by the wearable device, the weight information, the impedance information, and the first segmental body composition information of the measurement object into a body composition algorithm model corresponding to the measurement manner, to obtain the second segmental body composition information of the measurement object.

10. The method according to claim 1, wherein before the obtaining, by a body fat measurement device, measurement information of a measurement object, the method further comprises:

obtaining, by the body fat measurement device, contact information between the measurement object and the body fat measurement device, wherein the contact information comprises two-hand contact information and/or two-foot contact information between the body fat measurement device and the measurement object; and
determining, by the body fat measurement device, that the contact information of the measurement object meets a preset requirement.

11. The method according to claim 10, wherein the two-foot contact information comprises two-foot spacing information, two-foot pressure information, and standing location information; and
that the two-foot contact information meets a preset requirement comprises at least one of the following:

the two-foot spacing information indicates that a two-foot spacing of the measurement object is greater than a distance threshold;
the two-foot pressure information indicates that a pressure difference between two feet of the measurement object is not greater than a pressure difference threshold; and
the standing location information indicates that the two feet of the measurement object stand in a first specified region of the body fat measurement device.

12. The method according to claim 10, wherein the two-hand contact information comprises handle holding area information, handle rope length information, and handle holding location information; and
that the two-hand contact information meets a preset requirement comprises at least one of the following:

the handle holding area information indicates that a contact area between two hands of the measurement object and a handle of the body fat measurement device is greater than an area threshold;
the handle rope length information indicates that a handle rope length of the body fat measurement device falls within a preset rope length range; and
the handle holding location information indicates that the two hands of the measurement object hold in a second specified region of the handle of the body fat measurement device.

13. The method according to claim 10, wherein the body fat measurement device is a four-electrode body fat scale, and the contact information comprises the two-foot contact information; or the body fat measurement device is an eight-electrode body fat scale, and the contact information comprises the two-hand contact information and the two-foot contact information.

14. A body fat measurement device, comprising one or more processors, a memory, a sensor, a transceiver, and one or more computer programs, wherein

the one or more computer programs are stored in the memory, the one or more computer programs comprise instructions, and when the instructions are invoked and executed by the one or more processors, the body fat measurement device is enabled to perform the following steps:

controlling the sensor to obtain impedance information of a measurement object by measuring a first segment of the measurement object; controlling the sensor or the transceiver to obtain weight information of the measurement object; determining first segmental body composition information of the measurement object based on the weight information and the impedance information; and determining second segmental body composition information of the measurement object based on the weight information, the impedance information, and the first segmental body composition information of the measurement object.

15. The body fat measurement device according to claim 14, wherein the impedance information comprises first impedance and second impedance, the first impedance is obtained by the body fat measurement device by controlling the sensor to measure the first segment by using a measurement signal at a first frequency, the second impedance is obtained by the body fat measurement device by controlling the sensor to measure the first segment by using a measurement signal at a second frequency, and the first frequency is less than the second frequency.

16. The body fat measurement device according to claim 15, wherein the first frequency is 50 kHz, and the second frequency is 250 kHz.

17. The body fat measurement device according to claim 15 or 16, wherein the second segmental body composition information of the measurement object satisfies the following relational expression:

$$AM = \alpha_1 Z_1 + \alpha_2 Z_2 + \alpha_3 W + \alpha_4 H + \alpha_5 Age + \alpha_6 G + \alpha_7 LM + \alpha_0,$$

wherein
AM is the second segmental body composition information, $Z_1$ is the first impedance, $\alpha_1$ is a weighting coefficient of the first impedance, $Z_2$ is the second impedance, $\alpha_2$ is a weighting coefficient of the second impedance, W is the weight information of the measurement object obtained by the body fat measurement device by controlling the sensor or the transceiver, $\alpha_3$ is a weighting coefficient of the weight information, H is height information of the measurement object obtained by the body fat measurement device by controlling the transceiver, $\alpha_4$ is a weight-

ing coefficient of the height information, Age is age information of the measurement object obtained by the body fat measurement device by controlling the transceiver, $\alpha_5$ is a weighting coefficient of the age information, G is gender information of the measurement object obtained by the body fat measurement device by controlling the transceiver, a value of G is 1 or 0, $\alpha_6$ is a weighting coefficient of the gender information, LM is the first segmental body composition information, $\alpha_7$ is a weighting coefficient of the first segmental body composition information, and ao is a constant.

18. The body fat measurement device according to claim 14, wherein when the instructions are invoked and executed by the one or more processors, the body fat measurement device is further enabled to perform the following steps:
determining whole-body composition information of the measurement object based on the weight information, a weighting coefficient of the weight information, the impedance information, and a weighting coefficient of the impedance information; and determining third segmental body composition information of the measurement object based on the whole-body composition information, the first segmental body composition information, and the second segmental body composition information of the measurement object.

19. The body fat measurement device according to claim 18, wherein the body fat measurement device is a four-electrode body fat scale, the first segmental body composition information comprises left leg composition information and/or right leg composition information, the second segmental body composition information comprises left arm composition information and/or right arm composition information, and the third segmental body composition information comprises trunk composition information.

20. The body fat measurement device according to claim 18 or 19, wherein when the instructions are invoked and executed by the one or more processors, the body fat measurement device is further enabled to perform the following step:
determining a limb balance indicator of the measurement object based on the whole-body composition information, the first segmental body composition information, the second segmental body composition information, and the third segmental body composition information.

21. The body fat measurement device according to claim 20, further comprising a display, wherein
when the instructions are invoked and executed by the one or more processors, the body fat measurement device is further enabled to perform the following step:

controlling the display to display any one or more of the following: the first segmental body composition information, the second segmental body composition information, the third segmental body composition information, the whole-body composition information, the limb balance indicator, and an exercise suggestion corresponding to the limb balance indicator of the measurement object.

22. The body fat measurement device according to claim 14, wherein the body fat measurement device is a wearable device, the measurement information further comprises a measurement manner, and the measurement manner is corresponding to the first segment; and

when the instructions are invoked and executed by the one or more processors, the body fat measurement device is further enabled to perform the following step:

inputting the weight information, the impedance information, and the first segmental body composition information of the measurement object into a body composition algorithm model corresponding to the measurement manner, to obtain the second segmental body composition information of the measurement object.

23. The body fat measurement device according to claim 14, wherein when the instructions are invoked and executed by the one or more processors, the body fat measurement device is further enabled to perform the following steps:

controlling the sensor to obtain contact information between the measurement object and the body fat measurement device, wherein the contact information comprises two-hand contact information and/or two-foot contact information between the body fat measurement device and the measurement object; and

determining that the contact information of the measurement object meets a preset requirement.

24. The body fat measurement device according to claim 23, wherein the two-foot contact information comprises two-foot spacing information, two-foot pressure information, and standing location information; and

that the two-foot contact information meets a preset requirement comprises at least one of the following:

the two-foot spacing information indicates that a two-foot spacing of the measurement object is greater than a distance threshold;

the two-foot pressure information indicates that a pressure difference between two feet of the measurement object is not greater than a pressure difference threshold; and

the standing location information indicates that the two feet of the measurement object stand in a first specified region of the body fat measurement device.

25. The body fat measurement device according to claim 23, wherein the two-hand contact information comprises handle holding area information, handle rope length information, and handle holding location information; and

that the two-hand contact information meets a preset requirement comprises at least one of the following:

the handle holding area information indicates that a contact area between two hands of the measurement object and a handle of the body fat measurement device is greater than an area threshold;

the handle rope length information indicates that a handle rope length of the body fat measurement device falls within a preset rope length range; and

the handle holding location information indicates that the two hands of the measurement object hold in a second specified region of the handle of the body fat measurement device.

26. The body fat measurement device according to claim 23, wherein the body fat measurement device is a four-electrode body fat scale, and the contact information comprises the two-foot contact information; or the body fat measurement device is an eight-electrode body fat scale, and the contact information comprises the two-hand contact information and the two-foot contact information.

27. A computer-readable storage medium, wherein the computer-readable storage medium stores a computer program or instructions, and when the computer program or instructions are executed by a computer, the method according to any one of claims 1 to 13 is implemented.

Left arm (LA)

Right arm (RA)

Trunk (Tr)

Left leg (LL)

Right leg (RL)

FIG. 1

Body fat
measurement device    210

Terminal device    220

FIG. 2

210

Body fat measurement device  211

| Pressure measurement module | Information processing module  213 | Display module  215 |

213

215

Pressure
measurement
module

211

Information
processing module

Display module

212

214

216

Impedance
measurement
module

Storage module

Communication
module

FIG. 3

FIG. 4

FIG. 5

| | |
|---|---|
| A four-electrode body fat scale obtains measurement information of a detected person | 601 |
| The four-electrode body fat scale determines first segmental body composition information of the detected person based on weight information and impedance information | 602 |
| The four-electrode body fat scale determines second segmental body composition information of the detected person based on the weight information, the impedance information, and the first segmental body composition information of the detected person | 603 |

FIG. 6

FIG. 7

A four-electrode body fat scale obtains whole-body compositions and segmental body compositions of a detected person — 801

The four-electrode body fat scale determines a first balance indicator, and if the first balance indicator is greater than a first preset value, determines that arm muscle of the detected person is left/right unbalanced — 802

The four-electrode body fat scale determines a second balance indicator, and if the second balance indicator is greater than a second preset value, determines that leg muscle of the detected person is left/right unbalanced — 803

The four-electrode body fat scale determines a third balance indicator, and if the third balance indicator is less than a third preset value, determines that a content of the arm muscle of the detected person is low — 804

The four-electrode body fat scale determines a fourth balance indicator, and if the fourth balance indicator is less than a fourth preset value, determines that a content of the leg muscle of the detected person is low — 805

FIG. 8

Whole-body fat is 12.9
kilograms in weight

Left arm
0.6

Right arm
0.6

Trunk
5.2

Left leg
3.2

Right leg
3.3

Fat balance result: balanced

Whole-body muscle is 19.4
kilograms in weight

Left arm
1.9

Right arm
2.0

Trunk
4.5

Left leg
5.5

Right leg
5.5

Muscle balance result: There is less leg
muscle. Increasing leg exercise such as
weight-bearing deep squatting is suggested,
to obtain a more beautiful body shape.

FIG. 9

After detecting that a to-be-detected person stands on a scale body, a four-electrode body fat scale starts, and the four-electrode body fat scale measures weight information and impedance information of the to-be-detected person — 1001

The four-electrode body fat scale calculates whole-body compositions — 1002

The four-electrode body fat scale calculates leg compositions — 1003

The four-electrode body fat scale calculates arm compositions — 1004

The four-electrode body fat scale calculates trunk compositions — 1005

The four-electrode body fat scale calculates muscle balance indicators and fat balance indicators — 1006

The four-electrode body fat scale displays the whole-body compositions, the segmental body compositions, each balance indicator, and a related exercise suggestion of the to-be-measured person on a display interface of the four-electrode body fat scale — 1007

FIG. 10

1101: A four-electrode body fat scale obtains two-foot contact information of a detected person

1102: The four-electrode body fat scale determines whether the two-foot contact information of the detected person meets a preset two-foot requirement

No

Yes

1104: The four-electrode body fat scale prompts the detected person to adjust the two-foot contact information

1103: The four-electrode body fat scale starts measurement

FIG. 11

FIG. 12

1301: A four-electrode body fat scale determines that two feet of a detected person stand on the four-electrode body fat scale

1302:
The four-electrode body fat scale determines whether the two feet of the detected person are not in contact with each other

No → 1306: The four-electrode body fat scale prompts, by using a voice and/or an interface, the detected person not to make the two feet in contact with each other

Yes

1303:
The four-electrode body fat scale determines whether the two feet of the detected person stand in a balanced manner

No → 1307: The four-electrode body fat scale prompts, by using a voice, the detected person to keep the two feet standing in a balanced manner

Yes

1304:
The four-electrode body fat scale determines whether the detected person stands in a first specified region

No → 1308: The four-electrode body fat scale prompts, by using a voice and/or an interface, the detected person to make the two feet stand in the first specified region

Yes

1305: The four-electrode body fat scale starts measurement

FIG. 13

FIG. 14

A four-electrode body fat scale determines that a to-be-detected person stands on the body fat scale

↓

The four-electrode body fat scale displays a region circle of a first specified region

↓

The four-electrode body fat scale determines whether the detected person stands in the first specified region

No

↓

1. The four-electrode body fat scale prompts, by using a display interface, the to-be-detected person to stand in the specified measurement region

| ↑ | ↓ | → | ← |
|---|---|---|---|
| Locations of two feet of the detected person are roughly at a rear part of the specified region | Locations of two feet of the detected person are roughly at a front part of the specified region | Locations of two feet of the detected person are roughly at a left part of the specified region | Locations of two feet of the detected person are roughly at a right part of the specified region |
| ↖ | ↙ | ↗ | ↘ |
| Locations of two feet of the detected person are roughly at a right-rear part of the specified region | Locations of two feet of the detected person are roughly at a right-front part of the specified region | Locations of two feet of the detected person are roughly at a left-rear part of the specified region | Locations of two feet of the detected person are roughly at a left-front part of the specified region |

2. The four-electrode body fat scale prompts, by using a voice, the to-be-detected person to stand in the specified measurement region

FIG. 15

Body fat scale

Display interface

Specified region

FIG. 16

FIG. 17

(a)

(b)

(c)

(d)

(e)

(f)

FIG. 18

1901: An eight-electrode body fat scale obtains two-hand contact information of a detected person

1902: The eight-electrode body fat scale determines whether the two-hand contact information of the detected person meets a preset two-hand requirement

No

Yes

1904: The eight-electrode body fat scale prompts the detected person to adjust the two-hand contact information

1903: The eight-electrode body fat scale starts measurement

FIG. 19

Two-hand contact information

Handle holding area information

Handle rope length information

Handle holding location information

Display interface

Handle

Specified region

Specified region

FIG. 20

FIG. 21

Height

θ

Handle rope length

FIG. 22

An eight-electrode body fat scale determines that a to-be-detected person holds a handle

↓

The eight-electrode body fat displays region circles for a left-hand specified region and a right-hand specified region on a display interface

↓

The eight-electrode body fat scale determines whether a left hand and a right hand of the detected person respectively hold in the corresponding specified regions

No
↓

1: The eight-electrode body fat scale prompts, by using an interface, the to-be-detected person to adjust a holding location of the left hand to the corresponding specified region and/or a holding location of the right hand to the corresponding specified region

→      ←      →      ←

| A left-hand location is roughly at a left part of the left-hand specified region | A left-hand location is roughly at a right part of the left-hand specified region | A right-hand location is roughly at a left part of a third specified region | A right-hand location is roughly at a right part of a third specified region |

2. The eight-electrode body fat scale prompts, by using a voice, the to-be-detected person to place the left hand in the left-hand specified region and place the right hand in the right-hand specified region

FIG. 23

FIG. 24

Electrode 1

Electrode 2

Electrode 3

Electrode 4

(a)

(b)

FIG. 25

(a)

(b)

FIG. 26

A wearable device obtains measurement information of a detected person — 2701

The wearable device determines segmental body composition information of the detected person based on weight information, a measurement manner, and impedance information of the detected person — 2702

The wearable device obtains a second type of personal information of the detected person — 2703

The wearable device corrects segmental body composition information of the detected person based on the second type of personal information — 2704

FIG. 27

| Measurement information | First type of personal information | Second type of personal information |

Body composition calculation model

Interface display of a wearable device

FIG. 28

2904

2903

Transceiver

Sensor

2906

2901

2902

Processor

Memory

Computer
program

2905

FIG. 29

## INTERNATIONAL SEARCH REPORT

| International application No. |
|---|
| **PCT/CN2021/115493** |

**A.  CLASSIFICATION OF SUBJECT MATTER**

A61B 5/053(2021.01)i

According to International Patent Classification (IPC) or to both national classification and IPC

**B.  FIELDS SEARCHED**

Minimum documentation searched (classification system followed by classification symbols)

A61B 5

Documentation searched other than minimum documentation to the extent that such documents are included in the fields searched

Electronic data base consulted during the international search (name of data base and, where practicable, search terms used)

CNABS; CNTXT; VEN; OETXT: 阻抗, 频率, 体重, 节段, 上肢, 下肢, 两手, 两腿, 频率, 估计, 推断, 模型, impendance?, estimat+, deduc+, conclud+, upper, lower

**C.  DOCUMENTS CONSIDERED TO BE RELEVANT**

| Category* | Citation of document, with indication, where appropriate, of the relevant passages | Relevant to claim No. |
|---|---|---|
| Y | CN 110897640 A (NANJING MEDLANDER MEDICAL SCIENCE & TECHNOLOGY CO., LTD.) 24 March 2020 (2020-03-24) description, paragraphs [0005]-[0149], figures 1, 2 | 1-27 |
| Y | CN 1494869 A (SHIMADZU CORPORATION et al.) 12 May 2004 (2004-05-12) description, page 12 paragraph 14 to page 43 paragraph 1 | 1-27 |
| Y | CN 111481199 A (HUAWEI TECHNOLOGIES CO., LTD.) 04 August 2020 (2020-08-04) description, paragraph [0112] | 4, 17 |
| Y | CN 108601551 A (IMPEDIMED LIMITED) 28 September 2018 (2018-09-28) description paragraphs [0322]-[0474] | 10-13, 23-26 |
| A | US 2004260196 A1 (CHIH-CHENG LU) 23 December 2004 (2004-12-23) entire document | 1-27 |
| A | KR 20130027187 A (BIOSPACE CO., LTD.) 15 March 2013 (2013-03-15) entire document | 1-27 |
| A | US 6472617 B1 (SUNBEAM PRODUCTS INC.) 29 October 2002 (2002-10-29) entire document | 1-27 |

☐ Further documents are listed in the continuation of Box C.　☑ See patent family annex.

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search | Date of mailing of the international search report |
|---|---|
| **29 November 2021** | **06 December 2021** |

| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br><br>Telephone No. |
|---|---|

Form PCT/ISA/210 (second sheet) (January 2015)

## INTERNATIONAL SEARCH REPORT
### Information on patent family members

International application No.

**PCT/CN2021/115493**

| Patent document cited in search report | | | Publication date (day/month/year) | Patent family member(s) | | | Publication date (day/month/year) |
|---|---|---|---|---|---|---|---|
| CN | 110897640 | A | 24 March 2020 | None | | | |
| CN | 1494869 | A | 12 May 2004 | KR | 20030081188 | A | 17 October 2003 |
| | | | | JP | 2003299629 | A | 21 October 2003 |
| | | | | JP | 4105472 | B2 | 25 June 2008 |
| | | | | US | 2003216665 | A1 | 20 November 2003 |
| | | | | CN | 1271971 | C | 30 August 2006 |
| CN | 111481199 | A | 04 August 2020 | WO | 2021190291 | A1 | 30 September 2021 |
| CN | 108601551 | A | 28 September 2018 | EP | 3376951 | A1 | 26 September 2018 |
| | | | | EP | 3376951 | A4 | 14 August 2019 |
| | | | | US | 2020237254 | A1 | 30 July 2020 |
| | | | | JP | 2019500180 | A | 10 January 2019 |
| | | | | CN | 108471980 | A | 31 August 2018 |
| | | | | AU | 2016353538 | A1 | 31 May 2018 |
| | | | | US | 2018333071 | A1 | 22 November 2018 |
| | | | | US | 2018235508 | A1 | 23 August 2018 |
| | | | | US | 10653334 | B2 | 19 May 2020 |
| | | | | EP | 3340874 | A1 | 04 July 2018 |
| | | | | EP | 3340874 | A4 | 17 April 2019 |
| | | | | JP | 2021119994 | A | 19 August 2021 |
| | | | | JP | 2018538114 | A | 27 December 2018 |
| | | | | JP | 6878449 | B2 | 26 May 2021 |
| | | | | WO | 2017079794 | A1 | 18 May 2017 |
| | | | | AU | 2016354667 | A1 | 19 April 2018 |
| | | | | WO | 2017079793 | A1 | 18 May 2017 |
| US | 2004260196 | A1 | 23 December 2004 | None | | | |
| KR | 20130027187 | A | 15 March 2013 | KR | 101275101 | B1 | 17 June 2013 |
| US | 6472617 | B1 | 29 October 2002 | None | | | |

Form PCT/ISA/210 (patent family annex) (January 2015)

**EP 4 215 110 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202011193817